(19) Europäisches Patentamt
European Patent Office
Office européen des brevets

(11) Publication number: **0 259 779 B1**

(12) **EUROPEAN PATENT SPECIFICATION**

(45) Date of publication of patent specification: **10.08.94**

(51) Int. Cl.$^5$: **C07D 493/22**, A61K 31/365, A23K 1/17, A01N 43/90, //(C07D493/22,313:00,313:00, 311:00,307:00)

(21) Application number: **87112854.2**

(22) Date of filing: **03.09.87**

The file contains technical information submitted after the application was filed and not included in this specification

# (54) 23-Oxo (Keto) and 23-imino Derivatives of LL-F28249 Compounds.

(30) Priority: **12.09.86 US 907283**

(43) Date of publication of application:
**16.03.88 Bulletin 88/11**

(45) Publication of the grant of the patent:
**10.08.94 Bulletin 94/32**

(84) Designated Contracting States:
**AT BE CH DE ES FR GB GR IT LI LU NL SE**

(56) References cited:
**EP-A- 0 170 006**
**EP-A- 0 215 654**
**FR-A- 2 570 390**
**GB-A- 2 176 182**
**US-A- 4 289 760**

(73) Proprietor: **AMERICAN CYANAMID COMPANY**
**One Cyanamid Plaza**
**Wayne, NJ 07470-8426 (US)**

(72) Inventor: **Asato, Goro**
**10 Maddock Road**
**Titusville New Jersey 08560 (US)**
Inventor: **France, Donald John**
**RR No.1 Box 386A**
**Pennington New Jersey 08534 (US)**

(74) Representative: **Wächtershäuser, Günter, Prof. Dr.**
**Patentanwalt**
**Tal 29**
**D-80331 München (DE)**

**EP 0 259 779 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

The present invention relates to new 23-oxo (keto) and 23-imino derivatives of the compounds collectively defined as LL-F28249. These LL-F28249 antibiotics preferably are produced by the fermentation of the microorganism Streptomyces cyaneogriseus subspecies noncyanogenus, deposited in the NRRL under deposit accession no. 15773.

The LL-F28249 components α-λ are complex macrolides which have a 23-hydroxy substituent, as well as two other hydroxy groups. The selective oxidation of this 23-hydroxy group to a 23-oxo group and the subsequent derivatization of the oxo group to afford 23-imino derivatives are the subject matter of the present invention. These 23-oxo and 23-imino derivatives of the LL-F28249α-λ compounds are useful for the prevention, treatment or control of helmintic, ectoparasitic, insect, acarid and nematode infections and infestations in warm-blooded animals and agricultural crops.

### SUMMARY OF THE INVENTION

The present invention provides novel 23-oxo (keto) and 23-imino derivatives of the compounds designated LL-F28249 alpha through lambda.

The LL-F28249α-λ compounds have the following structural formula:

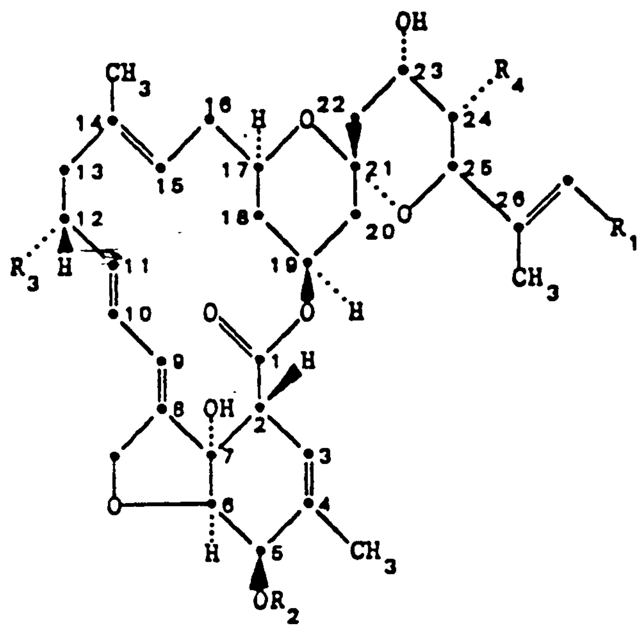

| Component | $R_1$ | $R_2$ | $R_3$ | $R_4$ |
|---|---|---|---|---|
| LL-F28249α | $CH(CH_3)_2$ | H | $CH_3$ | $CH_3$ |
| LL-F28249β | $CH_3$ | H | $CH_3$ | $CH_3$ |
| LL-F28249γ | $CH_3$ | $CH_3$ | $CH_3$ | $CH_3$ |
| LL-F28249ε | $CH(CH_3)_2$ | H | H | $CH_3$ |
| LL-F28249ζ | $CH_2CH_3$ | H | $CH_3$ | $CH_3$ |
| LL-F28249θ | $CH(CH_3)_2$ | H | $CH_3$ | $CH_2CH_3$ |
| LL-F28249ι | $CH(CH_3)_2$ | H | $CH_2CH_3$ | $CH_3$ |
| LL-F28249λ | $CH(CH_3)_2$ | $CH_3$ | $CH_3$ | $CH_3$ |

The compounds of the present invention are useful anthelmintics, ectoparasiticides, insecticides, acaricides and nematicides in treating, preventing or controlling such diseases in warm-blooded animals, such as poultry, cattle, sheep, swine, rabbits, horses, dogs, cats and human beings and agricultural crops.

Although these diseases have been recognized for years and therapies exist for the treatment and prevention of the diseases, the present invention provides novel compounds in the search for effective such therapy.

U.S. Patent 3,950,360, Aoki et al, April 13, 1976 discloses certain antibiotic substances obtained by culturing a Streptomyces microorganism, said compounds being useful as insecticides and acaricides. Further, an entire series of U.S. patents relates to certain compounds produced by the fermentation of Streptomyces avermitilis (U.S. Patent 4,171,314, Chabala et al, October 16, 1979; U.S. Patent 4,199,569, Chabala et al, April 22, 1980; U.S. Patent 4,206,205, Mrozik et al, June 3, 1980; U.S. Patent 4,310,519, Albers-Schonberg, January 12, 1982; U.S. Patent 4,333,925, Buhs et al, June 8, 1982). U.S. Patent 4,423,209, Mrozik, December 27, 1983 relates to the process of converting some of these less desirable components to more preferred ones. Finally, British Patent Application No. 2166436 A discloses antibiotics also.

The present compounds or the pharmaceutically and pharmacologically acceptable salts thereof exhibit excellent and effective treatment, prevention and/or control of these serious diseases of warm-blooded animals.

It is an object of the present invention, therefore, to provide novel 23-oxo and 23-imino derivatives of LL-F28249α-λ. It is a further object to provide a process for the preparation of these derivatives and to provide methods for preventing, treating or controlling endo and ectoparasitic (collectively parasitic), insect, nematode, acarid and helmintic diseases and infestation in warm-blooded animals and agricultural crops by providing compositions containing prophylactically, therapeutically or pharmaceutically-effective amounts of the present novel compounds.

These and other objects of the invention will become apparent by the more detailed description of the invention provided hereinbelow.

## DETAILED DESCRIPTION OF THE INVENTION

The LL-F28249 compounds which may act as precursors of the present compounds are represented by the following structural formula,

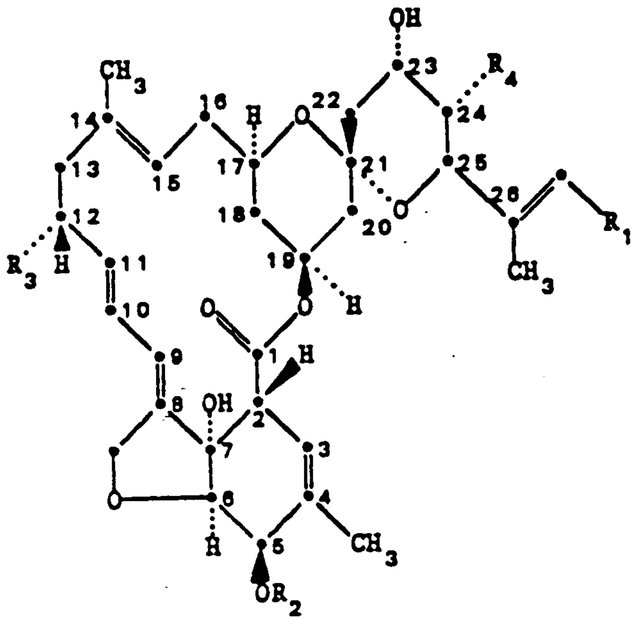

| Component | $R_1$ | $R_2$ | $R_3$ | $R_4$ |
|---|---|---|---|---|
| LL-F28249α | $CH(CH_3)_2$ | H | $CH_3$ | $CH_3$ |
| LL-F28249β | $CH_3$ | H | $CH_3$ | $CH_3$ |
| LL-F28249γ | $CH_3$ | $CH_3$ | $CH_3$ | $CH_3$ |
| LL-F28249ε | $CH(CH_3)_2$ | H | H | $CH_3$ |
| LL-F28249ζ | $CH_2CH_3$ | H | $CH_3$ | $CH_3$ |
| LL-F28249θ | $-CH(CH_3)_2$ | H | $CH_3$ | $CH_2CH_3$ |
| LL-F28249ι | $CH(CH_3)_2$ | H | $CH_2CH_3$ | $CH_3$ |
| LL-F28249λ | $CH(CH_3)_2$ | $CH_3$ | $CH_3$ | $CH_3$ |

wherein, $R_1$, $R_2$, $R_3$ and $R_4$ are defined by the above table.

The compounds of the instant invention are represented by the following structural formula:

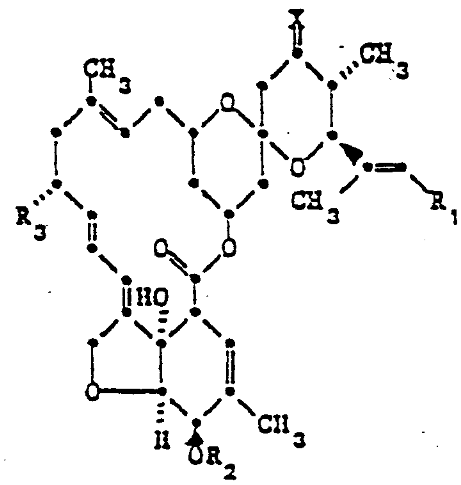

I

wherein $R_1$ is methyl, ethyl or isopropyl; $R_2$ is hydrogen, methyl, $C_1$-$C_4$ alkanoyl, methoxyacetyl, chloroacetyl, dichloroacetyl, trichloroacetyl, trifluoroacetyl; $R_3$ is hydrogen or methyl; X is $NOR_4$, or N-$NHR_5$; $R_4$ is hydrogen, $C_1$-$C_6$ alkyl, benzyl, allyl, propargyl, phenyl, $CH_2COO$-alkyl ($C_1$-$C_4$), N-($C_1$-$C_6$ alkyl)-carbamoyl, N-(allyl)carbamoyl, N-(propargyl)carbamoyl, N-(phenyl)carbamoyl, N-(chlorophenyl)carbamoyl, N-(dichlorophenyl)carbamoyl, N-(benzyl)carbamoyl, $C_1$-$C_6$ alkanoyl, chloroacetyl, methoxyacetyl, phenylacetyl, or chlorobenzoyl; $R_5$ is $C_1$-$C_6$ alkanoyl, formyl, $C_1$-$C_6$ alkyl, benzoyl optionally substituted with one or two halogens, $C_1$-$C_4$ alkyl groups, $C_1$-$C_4$ alkoxy groups or nitro groups;

and the pharmaceutically and pharmacologically acceptable salts thereof.

Preferably, $R_1$ is isopropyl; $R_2$ is hydrogen or methyl; $R_3$ is methyl; $R_4$ through $R_8$ are as described hereinabove; and X is $NOR_4$.

A preferred group of compounds of this invention is illustrated by formula I wherein $R_1$ is isopropyl; $R_2$ is hydrogen, $R_3$ is methyl; X is $NOR_4$;

$R_4$ is $C_1$-$C_3$ alkyl, N-($C_1$-$C_6$ alkyl)carbamoyl, N-(phenyl)carbamoyl, N-(4-chlorophenyl)carbamoyl, N-(benzyl)carbamoyl, $C_1$-$C_6$ alkanoyl, chloroacetyl, methoxyacetyl, benzoyl or chlorobenzoyl, and $R_6$ and $R_7$ are as described hereinabove.

A preferred group of compounds of structure (I) are the following:

wherein,

| | |
|---|---|
| $R_1$ | is isopropyl; |
| $R_2$ | is hydrogen or methyl; |
| $R_3$ | is methyl; |
| X | is $NOR_4$ or N-$NHR_5$; and |
| $R_4$ and $R_5$ | are the above-mentioned groups. |

Another preferred group of compounds of structure (I) are the following:

wherein,

| | |
|---|---|
| $R_1$ | is isopropyl; |
| $R_2$ | is hydrogen; |
| $R_3$ | is methyl; |
| X | is $NOR_4$ or N-$NHR_5$; |

and

$R_4$ and $R_5$ are the above-mentioned group.

The most preferred group of compounds of structure (I) are the following:

wherein,

| | |
|---|---|
| $R_1$ | is isopropyl; |
| $R_2$ | is hydrogen; |
| $R_3$ | is methyl; |
| X | is oxygen or $NOR_4$; and $R_4$ is |

$C_1$-$C_3$ alkyl,
N-($C_1$-$C_6$ alkyl)carbamoyl,
N-(phenyl)carbamoyl,
N-(4-chlorophenyl)carbamoyl,
N-(benzyl)carbamoyl,
$C_1$-$C_6$-alkanoyl, chloroacetyl, methoxyacetyl, benzoyl and chlorobenzoyl.

In preparing the compounds of the present invention, other hydroxy groups must be protected. Therefore, prior to the oxidxation of the 23-hydroxyl group to the 23-oxo or keto group is carried out, the 5-hydroxyl group is protected. Suitable protecting groups are trisubstituted silyl groups, such as t-butyldimethylsilyl and trimethylsilyl, or trisubstituted silyloxyacetyl groups, such as t-butyldimethylsilyloxy acetyl group. The protecting groups, however, are not limited to these groups since other useful protecting groups such as acyl and substituted acyl, such as acetyl, trifluoroacetyl, chloroacetyl, trichloroacetyl, phenoxyacetyl and the like, are also useful in the present process.

One of the preferred protecting groups is t-butyldimethylsilyl. This group is attached to the 5-hydroxyl group by reacting an unprotected 5-hydroxy F-28249 compound with t-butyldimethylsilyl chloride in the presence of a base, such as imidazole, pyridine, 4-dimethylaminopyridine, triethylamine and the like, in an aprotic solvent such as methylene chloride, toluene, ethylacetate, tetrahydrofuran, ethylenedichloride and the like. The reaction is stirred at a temperature of about 0°C to 30°C, and the reaction is complete in several hours, depending on the temperature of the reaction. The completion of the reaction is usually monitored by high performance liquid chromatography (HPLC) using reverse phase on a Whatman Partisil CCS/$C_8$ rapid analysis column.

Another preferred protecting group is t-butyldimethylsilyloxy acetyl group. This group is attached to the 5-hydroxyl group by combining the unprotected F-28249 compound in an aprotic solvent such as methylene chloride, toluene, ethyl acetate, tetrahydrofuran, ethylenedichloride and the like, containing a tertiary amine, such as pyridine or triethylamine, and adding the protecting agent in the form of an acid halide. The reaction is conducted at a temperature of about 0°C to 30°C and is monitored by HPLC for completion.

The 23-hydroxyl group of the protected F-28249 compound then is oxidized to the 23-oxo (or keto) group by using oxidizing agents such as pyridinium dichromate, pyridinium chlorochromate, chromic acid-dimethylpyrazole, acetic anhydride/dimethylsulfoxide, trifluoroacetic anhydride/dimethylsulfoxide, N-chlorosuccinimide/dimethylsulfoxide, oxalyl chloride/dimethylsulfoxide and the like. The reaction is carried out at dry-ice bath temperatures (about -78°C) to room temperature (about 25°C) and is complete in about 1 to 24 hours, depending on the rate of oxidation, which is monitored by HPLC. The dimethylsulfoxide oxidation procedures are carried out in the presence of triethylamine or diisopropylethylamine. Solvents such as methylene chloride, ethylenedichloride, dimethylformamide, dimethylsulfoxide and the like are used. In using oxalyl chloride/dimethylsulfoxide in the presence of triethylamine, it is advantageous to add molecular sieves to the reaction mixture to increase the yield. The oxidation may also be carried out by soil microorganisms using 100 mg to 10 g of a 23-hydroxy compound per liter of unsterilized soil at 20°C to 30°C. The oxidized 23-keto compound is extracted from the soil by a solvent such as acetone, methanol or ethanol.

The silyl protecting group is removed by stirring a protected 23-keto LL-F28249 compound in a lower alkanol such as methanol at 0° to room temperature for about 0.5 hour to an hour in the presence of an acid such as p-toluenesulfonic acid. If the protecting group is a silyloxyacetyl group, the silyl group is removed with acid as described above, and the hydroxyacetyl group is cleaved with an equivalent of base such as sodium methoxide in methanol at 0° to room temperature in 0.5 hour to several hours. The silyloxyacetyl group may also be removed in one step by treatment with sodium methoxide at room temperature until the reaction is complete. Similarly, other acyl protecting groups are removed by base treatment.

The imino derivatives of the 23-oxo compounds are readily prepared by standard techniques such as procedures described by S. M. McElvain in The Characterization of Organic Compounds, published by MacMillan Company, New York, 1953, pages 204-205.

Typically, a 23-oxo compound is stirred in alcohol, such as methanol or ethanol, or dioxane in the presence of acetic acid and an excess of the amino derivatizing agent, such as hydroxylamine hydrochloride, 0-methylhydroxylamine hydrochloride, semicarbazide hydrochloride and the like along with an equivalent amount of sodium acetate, at room temperature to 50°C. The reaction is usually complete in several hours to several days at room temperature but can be readily speeded by heating.

The O-acyloximes or carbamoylated oximes are prepared by treating the oximes of structure (I) with acid anhydrides or isocyanates to afford (I), wherein $R_4$ is $C_1$-$C_6$ alkanoyl, chloroacetyl, methoxyacetyl,

phenylacetyl, benzoyl, chlorobenzoyl, N-($C_1$-$C_4$ alkyl)carbamoyl, N-(allyl)carbamoyl, N-(propargyl)-carbamoyl, N-(phenyl)carbamoyl, N-(chlorophenyl)carbamoyl, N-(dichlorophenylcarbamoyl or N-(benzyl)-carbamoyl. The reactions are conducted in inert solvents, such as methylene chloride, ethylenedichloride or dioxane, in the presence of a tertiary amine, such as triethylamine or dissopropylethylamine. Generally, the reactions are conducted from 0 °C to room temperature, but if the reactions are sluggish, heat is applied. An equivalent to a slight excess of the acid anhydride is used to avoid reaction at the 5-hydroxy group.

The novel compounds of the present invention have significant activity as anthelmintics, ectoparasiticides, insecticides, nematicides and acaricides in human and animal health areas and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oestophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris and Paracaris. Certian of these, such as Nematodirus, Cooperia, and Oesphagostomum primarily attack the intestinal tract while others, such as Haemonchus and Ostertagia, are most prevalent in the stomach. Still others such as Dictyocaulus are found in the lungs. Also, other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs, and if left untreated, may result in death of the infected host. The 23-oxo or -imino derivatives of the LL-F28249 compounds of this invention unexpectedly have high activity against these parasites. Additionally, they also are active against Dirofilaria in dogs, Nematospiroides, Syphacia, Aspiculuris in rodents, arthropod ectoparasites such as ticks, mites, lice, fleas, blowfly of animals and birds, the ectoparasite Lucilia sp. of sheep, biting insects and migrating dipterous larvae such as Hypoderma sp. in cattle, Gastrophilus in horses and Cuterebra sp. in rodents.

The compounds of the present invention also are useful in treating, preventing or controlling parasites which infect human beings, as well. The most common genera of parasites of the gastrointestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunculus and extra-intestinal stages of the intestinal worms Strongyloides and Trichinella. The present compounds also are of value against arthropods parasitizing man, biting insects and other dipterous pests causing annoyance to man.

These compounds further are active against household pests such as the cockroach, Blattella sp., clothes moth, Tineola sp., carpet beetle, Attagenus sp., and the housefly Musca domestica.

Insect pests of stored grains such as Tribolium sp., Tenebrio sp., and of agricultural plants such as spider mites (Tetranycus sp.), southern army worms, tobacco budworms, boll weevils, aphids (Acyrthiosiphon sp.), migratory orthopterans such as locusts and immature stages of insects living on plant tissue are controlled by the present compounds as well as the control of soil nematodes and plant parasites such as Meloidogyne sp., which may be of importance in agriculture.

The compounds of the present invention may be administered orally or parenterally for animal and human usage, while they may be formulated in liquid or solid form for agricultural use. Oral administration may take the form of a unit dosage form such as a capsule, bolus or tablet, or as a liquid drench where used as an anthelmintic for animals.

The animal drench is normally a solution, suspension or dispersion of the active compound, usually in water, together with a suspending agent such as bentonite and a wetting agent or like excipient. Generally, the drenches also contain an antifoaming agent. Drench formulations generally contain about 0.001% to 0.5%, by weight, of the active compound. Preferred drench formulations contain about 0.01% to 0.1% by weight.

Capsules and boluses comprise the active ingredient admixed with a carrier vehicle such as starch, talc, magnesium stearate or di-calcium phosphate.

Where it is desired to administer the 23-oxo or 23-imino derivatives of LL-F28249 in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with

respect to their total weight and content of the active compound depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the active compounds of the present invention may be administered to animals parenterally, such as by intraruminal, intramuscular, intratracheal, or subcutaneous injection. In such an event, the active compound is dissolved or dispersed in a liquid carrier vehicle.

For parenteral administration, the active compound is suitable admixed with an acceptable vehicle, preferably of the vegetable oil variety such as peanut oil, cotton seed oil and the like. Other parenteral vehicles such as organic preparations using solketal, propylene glycol, glycerol formal, and aqueous parenteral formulation also are used. The active 23-oxo or -imino compound or compounds of the present invention are dissolved or suspended in the parenteral formulation for administration. Such formulations generally contain about 0.005% to 5%, by weight, of the active compound.

Although the compounds of the present invention are primarily uses in the treatment, prevention or control of helminthiasis, they also are useful in the prevention and treatment of diseases caused by other parasites. For example, arthropod parasites such as ticks, lice, fleas, mites and other biting insects in domesticated animals and poultry are controlled by the present compounds. These compounds also are effective in treatment of parasitic diseases that occur in other animals including human beings. The optimum amount to be employed will, of course, depend upon the particular compound employed, the species ofanimal to be treated and the type and severity of parasitic infection or infestation. Generally, the amount useful in oral administration of these novel compounds is about 0.001 mg to 10 mg per kg of animal body weight, such total dose being given at one time or in divided doses over a relativelyshort period of time (1-5 days). The preferred compounds of the invention give excellent control of such parasites in animals by administering about 0.025 mg to 3 mg per kg of animal body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of the animal's feed, or dissolved or suspended in the drinking water, compositions are provided in which the active compound or compounds are intimately dispersed in an inert carrier or diluent. An inert carrier is one that will not react with the active component and that will be administered safely to animals. Preferably, a carrier for feed administration is one that is, or may be, an ingredient of the animal ration.

Suitable compositions include feed premixes or supplements in which the active compound is present in relatively large amounts, wherein said feed premixes or supplements are suitable for direct feeding to the animal or for addition to the feed either directly or after an intermediate dilution or blending step.

Typical carriers or diluents suitable for such compositions include distillers' dried grains, corn meal, citrus meal, fermentation residues, ground oyster shells, wheat shorts, molasses solubles, corn cob meal, edible bean mill feed, soya grints, crushed limestone and the like. The active compounds are intimately dispersed throughout the carrier by methods such as grinding, stirring, milling or tumbling. Compositions containing about 0.005% to 2.0%, by weight, of the active compound are particularly suitable as feed premixes.

Feed supplements, which are fed directly to the animal, contain about 0.0002% to 0.3%, by weight, of the active compounds. Such supplements are added to the animal feed in an amount to give the finished feed the ccncentration of active compound desired for the treatment, prevention and/or control of parasitic diseases. Although the desired concentration of active compound will vary depending upon the factors previously mentioned as well as upcn the particular derivative employed, the compounds of this invention are usually fed at concentrations of about 0.00001% to 0.02% in the feed in order to achieve the desired antiparasitic result.

The compounds also may be administered by pouring on the skin of animals via a solution. Generally, the active compounds are dissolved in a suitable inert solvent, such as dimethylsulfoxide, propylene glycol of the like, alternatively in combination of solvents, for the pour-on administration.

The compounds of this invention also are useful in combating agricultural pests that inflict damage upon growing or stored crops. The present compounds are applied, using known techniques such as sprays, dusts, emulsions and the like, to the growing or stored crops to effect protection from such agricultural pests.

The present invention is illustrated by the following examples which are illustrative of said invention and not limitative thereof.

EXAMPLE 1

## 5-0-t-Butyldimethylsilyl-LL-F28249α

In 500 mL of $CH_2Cl_2$, 70 g of LL-F28249α is stirred with 82.04 g imidazole at 20°C under $N_2$ atmosphere. Then, 43 g of t-butyldimethylsilyl chloride in 400 mL of $CH_2Cl_2$ is added over 5 minutes. After an hour, the reaction is assayed for completion by high performance liquid chromatography (HPLC), using 50% $CH_3CN$/50% $H_2O$ in a curved gradient mode over 10 minutes on a Whatman Partisil CCS/$C_8$ rapid analysis column at 1 mL/min flowrate. Another 3 g of t-butyldimethylsilyl chloride is added, and after 3 hours the composition is 92.3% product, 0.3% LL-F28249α and 1.16% disilylated material. The mixture is diluted with $CH_2Cl_2$ and poured into 2 L of $H_2O$. The $CH_2Cl_2$ layer is separated. The aqueous portion is extracted with 2 L of $CH_2Cl_2$, and the combined organic layers are dried ($Na_2SO_4$). The $CH_2Cl_2$ is evaporated in vacuo to afford 116 g of the title compound that is identified by mass spectrometry and nuclear magnetic resonance (NMR) spectrometry.

EXAMPLE 2

## 5-0-t-Butyldimethylsilyl-23-oxo-LL-F28249α

In 5 L of dry $CH_2Cl_2$, 116 g of 5-0-t-butyldimethylsilyl LL-F28249α is stirred under $N_2$, and 540 g of NaOAc is added at 22°C, followed by addition of 172.5 g of pyridinium chlorochromate (PCC). After 1 hour, an additional 15 g of PCC is added since the reaction is incomplete by HPLC analysis. After 2 hours, another 10 g of PCC is added, and the reaction is stirred for a total of 5 hours. The mixture is poured into 6 L of ice-water mixture, and the $CH_2Cl_2$ is separated. The aqueous layer is extracted with $CH_2Cl_2$, and the combined $CH_2Cl_2$ layers are washed with water and dried ($Na_2SO_4$). The $CH_2Cl_2$ is evaporated in vacuo to afford 197.8 g of crude product, which is dissolved in 2 L of $Et_2O$ and filtered. The $Et_2O$ solution is washed with water (2 x 1000 mL), dried ($Na_2SO_4$) and evaporated to dryness to give 60 g of the title compound which is identified by mass spectrometry and NMR spectroscopy.

The pyridinium chlorochromate substituted with pyridinium dichromate in the above procedure also affords the title compound.

EXAMPLE 3

23-Oxo-LL-F28249α

In 1.5 L of MeOH, 60 g of 5-0-t-butyldimethylsilyl-23-oxo-LL-F28249α is dissolved by warming, and at 0°C, 30 g of p-toluenesulfonic acid in 300 mL of MeOH is added. The mixture is stirred for 3 hours and poured into 6 L of saturated $NaHCO_3$ solution in 6 L of $H_2O$. After stirring, the mixture is extracted with 4 L of EtOAc, and the layers are separate. The aqueous layer is saturated with NaCl and extracted with 2 x 6 L of EtOAc. The first EtOAc layer is washed with saturated NaCl solution, combined with the other EtOAc extracts and dried ($Na_2SO_4$). The EtOAc is evaporated in vacuo to afford 148.1g of dark residue. The crude material is then chromatograpned by HPLC on 1200 g of $SiO_2$ using 1% isopropanol in $CH_2Cl_2$ to elute and monitored by an ultraviolet detector/254 nM filter. Fractions 39-42 are combined and evaporated to dryness to afford 12.65 g of the title compound which analyzes as follows:

| | | |
|---|---|---|
| Anal. Calcd for $C_{36}H_{50}O_8$: | C, 70.79; | H, 8.25 |
| Found: | C, 70.33; | H, 8.31 |

The title compound is further identified by mass spectrometry and NMR spectroscopy.

EXAMPLES 4 AND 5

23-O-Methyloxime-LL-F28249α

In 930 mL of dry dioxane at room temperature, 70 g of 23-oxo-LL-F28249α, 11.8 g of NaOAc, 11.8 g of $CH_3ONH_2$•HCl and 2.1 mL of HOAc are added. The mixture is stirred under $N_2$ for 3 days, and after no starting material is detected by HPLC, 650 mL of dioxane is evaporated in vacuo. The residue is poured into 5 L of $H_2O$, and the product is extracted with $CH_2Cl_2$ (4 x 2 L). The combined extracts are washed with $H_2O$, dried ($Na_2SO_4$) and evaporated to dryness. The residue is dissolved in 1500 mL of $Et_2O$, and the solution is washed with $H_2O$, dried ($Na_2SO_4$) and evaporated to dryness. This gives 11.84 g of the title compound, which is identified by mass spectrometry and NMR spectroscopy. It also analyzes as follows:

| | | | |
|---|---|---|---|
| Anal. Calcd for $C_{37}H_{53}O_8N$•1.5 $H_2O$: | C, 66.64; | H, 8.46; | N, 2.10 |
| Found: | C, 66.82; | H, 8.13; | N, 2.32 |

The title compound is acetylated with excess acetic anhydride in methylene chloride at room temperature to afford 5-acetyl-23-0-methyloxime-LL-F28249α, which is identified by mass spectrometry and NMR spectroscopy after chromatographic purification.

EXAMPLE 6

23-Oxime-LL-F28249α

In 5 mL of dioxane, 62 mg of 23-oxo-LL-F28249α is stirred with 49 mg of $NH_2OH$•HCl, 50 mg of NaOAc and 10 µl of HOAc for 23 hours under $N_2$ atmosphere. The mixture is poured into 250 mL each of $H_2O$ and $CH_2Cl_2$, and the $CH_2Cl_2$ layer is separated. The aqueous layer is extracted with 50 mL of $CH_2Cl_2$, and the combined $CH_2Cl_2$ solutions are washed with $H_2O$, dried ($Na_2SO_4$) and evaporated to dryness to afford 8.8 mg of residue. This material is purified on a preparative layer plate (silica gel) using 20% MeOH in $CH_2Cl_2$ to afford the title compound which is identified by mass spectrometry and NMR spectroscopy.

EXAMPLE 7

23-[O-(Methylcarbamoyl)oxime]-LL-F28249α

In 5 mL of $Et_2O$, 27.2 mg of 23-oxime-LL-F28249α is stirred under $N_2$ with 10 µl of $Et_3N$ and 50 µL of methyl isocyanate for 17 hours at room temeprature. The ether is evaporated, and the residue is purified on a preparative chromatography plate (silica gel) using 20% MeOH in $CH_2Cl_2$ to afford the title compound, which is identified by mas spectrometry and NMR spectroscopy.

EXAMPLES 8-20

The following 23-0-substituted oxime-LL-F28249α compounds are prepared by the method of Example 4 using the appropriate 0-substituted hydroxylamines in place of methoxyamine:

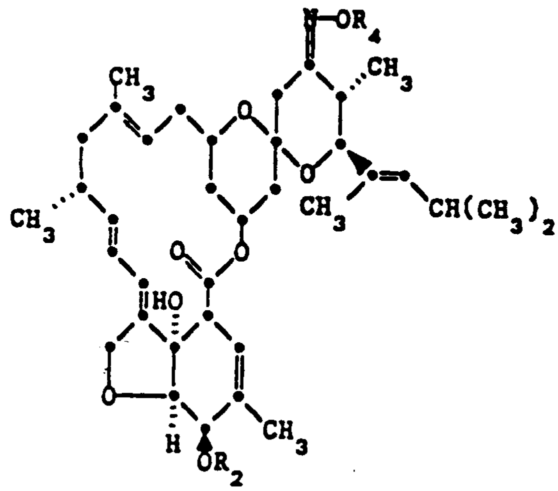

| $R_2$ | $R_4$ |
|---|---|
| H | Ethyl |
| H | n-Hexyl |
| H | i-Propyl |
| H | Benzyl |
| H | Phenyl |
| H | Allyl |
| H | Propargyl |
| H | $CH_2COO$-t-Butyl |
| H | $CH_2COO$-Ethyl |
| $CH_3$ | Ethyl |
| $CH_3$ | Benzyl |
| $CH_3CO$ | Ethyl |
| $ClCH_2CO$ | Ethyl |

EXAMPLE 21

## 5-0-t-Butyldimethylsilyl-23-oxime-LL-F28249α

In the manner described in Example 5, 5-0-t-butyldimethylsilyl-23-oxime-LL-F28249α is prepared and purified by chromatography over silica gel.

EXAMPLES 22-32

23-[0-Subsituted-carbamoyl)oxime]-LL-F28249α

The following 23-0-substituted-carbamoyl)oximes of LL-F28249α are prepared. by the method of Example 7 by using the appropriate isocyanates:

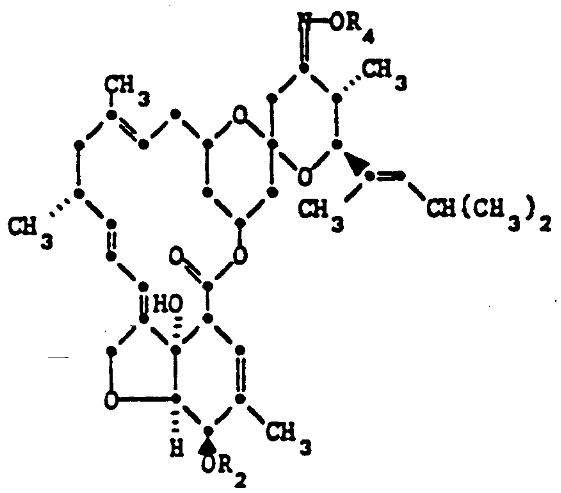

| $R_2$ | $R_4$ |
|---|---|
| H | $C_2H_5NH-CO$ |
| H | $n-C_6H_{13}NH-CO$ |
| H | $i-C_3H_7NH-CO$ |
| H | Benzyl-NH-CO |
| H | Phenyl-NH-CO |
| H | 4-Chlorophenyl-NH-CO |
| H | Allyl-NH-CO |
| H | Propargyl-NH-CO |
| $CH_3$ | $C_2H_5NH-CO$ |
| $CH_3$ | Benzyl-NH-CO |
| H | 3,4-Dichlorophenyl-NHCO |

EXAMPLE 33

23-[0-Acetyl)oxime]-LL-F28249α

In 1 mL of $CH_2Cl_2$, 60 mg of 23-oxime-LL-F28249α is stirred with 6 μL of triethylamine at 0 °C, and 5 mL of a solution ccntaining 244.4 mg of acetic anhydride in 100 mL of $CH_2Cl_2$ is added. The reaction mixture is stirred at 0 °C for 24 hours and evaporated to dryness in vacuo. The residue is dissolved in 50 mL of $CH_2Cl_2$ and washed with 30 mL of $H_2O$. The solution is dried ($Na_2SO_4$) and evaporated to dryness to afford 53.2 mg of the title compound, which i identified by NMR spectroscopy amd mass spectrometry.

EXAMPLE 34-41

By the procedure of Example 33, the following 23-[0-(substituted)oximes] of LL-28249 are prepared by using the appropriate acid anhydrides.

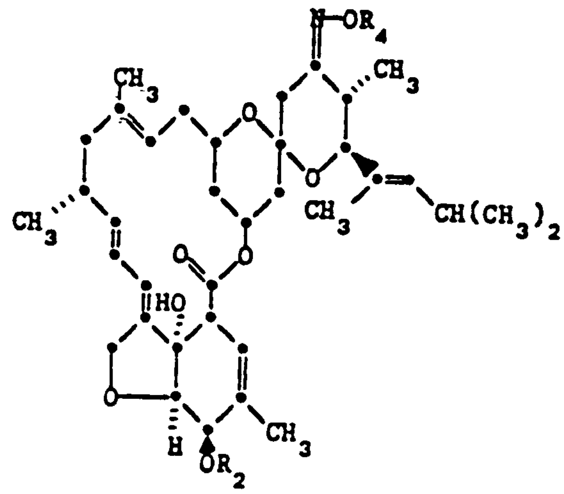

| $R_2$ | $R_4$ |
|---|---|
| H | $ClCH_2CO$ |
| H | $CH_3OCH_2CO$ |
| H– | $n-C_4H_9CO$ |
| H | $i-C_4H_9CO$ |
| H | $C_6H_5CO$ |
| H | $C_6H_5CH_2CO$ |
| $CH_3$ | $CH_3OCH_2CO$ |
| H | Phenylacetyl |
| H | $C_6H_5OCH_2CO$ |

EXAMPLES 42

5-0-Acetyl-23-Oxo-LL-F28249α

In 2.5 mL of $CH_2Cl_2$, 61 mg of 23-oxo-LL-F28249α is stirred with 49 mg of 4-dimethylaminopyridine, and 52 mg of diisopropylethylamine and 32 mg of acetyl chloride in 1 mL of $CH_2Cl$ is added at 0°C. The mixture is stirred at 0-5°C for 1.5 hours and poured into ice/$H_2O$, and the aqueous mixture is made acidic with 1:1 aqueous HCl. The mixture is then extracted with 3 x 2 mL of $CH_2Cl_2$, and the extracts are dried ($MgSO_4$) and evaporated to dryness. The crude product is dissolved in acetone and applied on a preparative silica gel plate and chromatographed in 20 $CH_2Cl_2$/l EtOAc (vol/vol). The title compound is recovered by extraction with 10% MeOH in $CH_2Cl_2$ and identified by mass spectrometry and NMR spectroscopy.

EXAMPLE 43

5-0-Acetyl-23-methoxime-LL-F28249α

In the manner described in Example 4 and 5, 5-0-acetyl-23-oxo-LL-F28249α is reacted with $MeONH_2 \cdot HCl$ to afford the title compound, which is identified by mass spectrometry and NMR spectroscopy.

EXAMPLES 44-50

5-0-Acyl-23-oxo-LL-F28249α

Using the procedure of Example 42, the following 5-0-acyl-23-oxo-LL-F28249α compounds are prepared using the requisite acid chlorides.

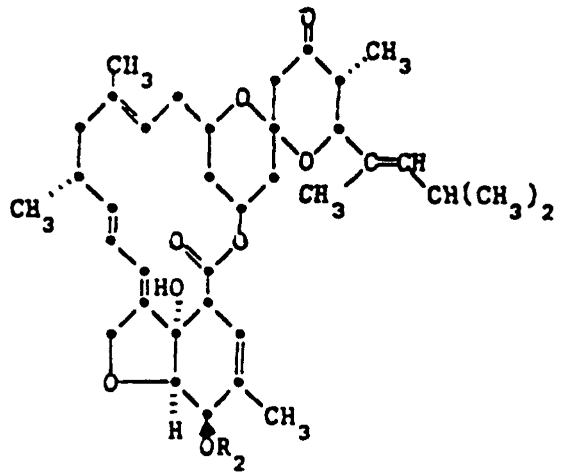

| $R_2$ |
|---|
| $CH_3OCH_2CO$ |
| $ClCH_2CO$ |
| $(CH_3)_2CH-CO$ |
| $\underline{n}-C_3H_7CO$ |
| $Cl_3C-CO$ |
| $CF_3CO$ |
| $Cl_2HCCO$ |
| $C_6H_5OCH_2CO$ |

EXAMPLES 51-57

5-0-Acyl-23-0-Substituted-Oxime-LL-F28249α

By the procedure of Examples 4 and 5, the following oximes are prepared by reacting the 5-0-acyl-23-oxo-LL-F28249α with the appropriate 0-substituted hydroxylamine.

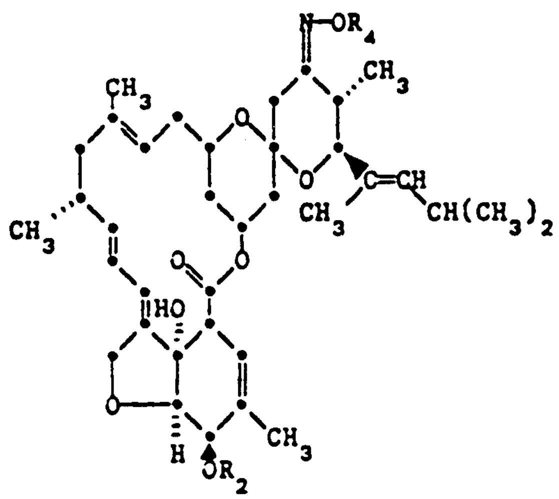

| $R_2$ | $R_4$ |
|---|---|
| $ClCH_2CO$ | $CH_3$ |
| $CH_3OCH_2CO$ | $CH_3$ |
| $CH_3OCH_2CO$ | $C_2H_5$ |
| $Cl_3CCO$ | $CH_3$ |
| $CF_3CCO$ | $CH_3$ |
| $CH_3CO$ | $CH_2COOC_2H_5$ |
| $CH_3OCH_2CO$ | Benzyl |
| $C_6H_5OCH_2CO$ | Methyl |

EXAMPLE 58

23-Oxo-LL-F28249α

A solution containing 0.32 g of oxalyl chloride in 5 mL of $CH_2Cl_2$ is cooled and stirred with 0.71 g of Type 4A molecular sieves in a dry-ice/acetone bath, and a mixture of 0.4 g of dimethylsulfoxide (DMSO) in 2 mL of $CH_2Cl_2$ is added slowly with stirring under $N_2$ atmosphere. Subsequently, 0.83 g of 5-0-$\underline{t}$-butyldimethylsilyl-LL-F28249α in 8 mL of $CH_2Cl_2$ is added dropwise over 10 minutes. After 0.5 hours, 1.6

mL of triethylamine is added dropwise, and the mixture is allowed to rise to room temperature (about 25 °C) over an hour. The mixture is poured into an ice-$H_2O$ mixture, and the aqueous mixture is extracted with 3 x 10 mL of $Et_2O$. The $Et_2O$ layers are washed with $H_2O$ (5 x 5 mL) and dried ($MgSo_4$). Ether is removed, and the residue is dissolved in 15 mL of MeOH. The MeOH solution is stirred at 0 °C with 0.3 g p-toluenesulfonic acid for 3 hour and poured into 60 mL of saturated $NaHCO_3$ and 60 mL of $H_2O$. The aqueous mixture is stirred with NaCl until it is saturated and extracted with EtOAc (3 x 40 mL). The combined EtOAc layers are dried ($MgSO_4$) and evaporated to dryness to afford the title compound that is identical with the material obtained by the method of Example 3.

EXAMPLE 59

23-Oxo-LL-F28249λ

Using the procedure of Example 2, LL-F28249λ is converted to the title compound 23-oxo-LL-F28249λ, which is purified by chromatography on silica gel and identified by mass spectrometry and NMR spectroscopy.

EXAMPLES 60-61

23-Oxo-LL-F28249β

By the procedure of Example 1, LL-F28249β is protected with 5-t-butyldimethylsilyl chloride, and the protected product is oxidized in manner described in Example 2. Deprotection of the 5-silyl group is accomplished by the procedure of Example 3 to afford the title compound which is identified by mass spectrometry and NMR spectroscopy.

Similar 23-oxo-LL-F28249λ is prepared from LL-F28249λ.

EXAMPLE 62

23-Semicarbazone-LL-F28249α

In 6 mL of dry dioxane under $N_2$ atmosphere, 60 mg of 23-oxo-LL-F28249α is stirred with 56.4 mg of NaOAc, 77 mg of semicarbazide hydrochloride and 18 µL of HOAc for 6 days at room temperature. The mixture is poured on ice and mixed with 100 mL each of $H_2O$ and $CH_2Cl_2$. The $CH_2Cl_2$ layer is separated, and the aqueous layer extracted with 50 mL of $CH_2Cl_2$. The combined extracts are washed with 75 mL of $H_2O$ and evaporated to dryness. The residue is dissolved in 100 mL of $Et_2O$, and the solution is washed with 8 x 50 mL of $H_2O$, dried ($Na_2SO_4$) and evaporated to dryness to afford 47.5 mg of the title compound.

EXAMPLES 63-68

In the same manner the following semicarbazones and thiosemicarbazone compounds are prepared by the procedure of Example 62.

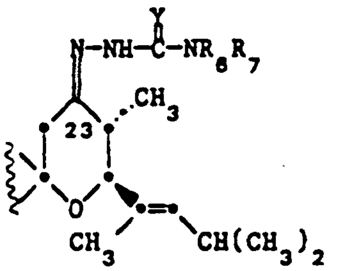

(partial structure of LL-F28249α)

| Y | $R_6$ | $R_7$ |
|---|---|---|
| S | H | H |
| S | $CH_3$ | $CH_3$ |
| O | $CH_3$ | H |
| O | $CH_3$ | $CH_3$ |
| O | $n-C_4H_9$ | H |
| O | $i-C_3H_7$ | H |
| O | $C_6H_5$ | H |
| *S | $C_6H_5$ | H |

*=methanol used as solvent in this preparation

EXAMPLES 69-73

23-(2-Carbomethoxyhydrazone)-LL-F28249α

In 15 mL of MeOH, 50 mg of 23-oxo-LL-F28249α is stirred with 25 mg of methyl carbazate in the presence of 10 μL of HOAc. After 3 days, the mixture is poured on ice and diluted with $H_2O$. The aqueous phase is saturated with salt and extracted with $CH_2Cl_2$ several times. The $CH_2Cl_2$ extracts are dried ($Na_2SO_4$) and evaporated to dryness. The residue is purified by chromatography on silica gel using 2% isopropanol in $CH_2Cl_2$ as eluent to afford the title compound.

Similarly the 2-carbethoxyhydrazone and 2-carbobutoxyhydrazones are prepared using the appropriate carbazates. Also, 1-methylhydrazine is similarly reacted with the above ketone to afford 23-(1-methylhydrazone)-LL-F28249α, while use of acethydrazide affords 23-acethydrazone-LL-F28249α.

Following the above procedure but substituting formic acid hydrazide for semicarbazide hydrochloride yields 23-(formylhydrazone)-F28249α in the form of a white solid.

EXAMPLE 74

23-[O-(ethoxymethyl)oxime]-F28249α

A suspension of 40 mg 23-oximino-LL-F28249α, 30.2 mg chloromethylethylether, 44.2 mg potassium carbonate and 2 mL DMF is stirred at room temperature for 36 hours. The mixture is diluted with water and extracted twice with 50 mL of ether. The combined ether layers are washed with 50 mL of water and 50 mL of brine, dried ($MgSO_4$), and evaporated. The residue is purified by chromatography on silica gel using 1.0 to 1.5% isopropanol an $CH_2Cl_2$ as eluent to afford the title compound in the form of a white glass.

EXAMPLE 75

23-[(p-tolylsulfonyl)hydrazone]-F28249α

A solution of 25.5 mg of 23-oxo-LL-F28249α, 7.7 mg of toluenesulfonylhydrazine and 1 mL ethanol is at reflux for 30 minutes. An additional 5.0 mg of the hydrazone is added and heating continued for an additional 30 minutes. The mixture is diluted with 5 mL of ether and is extracted with 2 mL of $H_2O$ followed by 1 mL of brine containing 3 drops of acetic acid. The combined aqueous layers are washed with 3 mL of ether. The combined ether solutions are dried ($MgSO_4$) and evaporated. The residue is purified by silica gel chromatography on silica gel using 1.5% isopropanol in $CH_2Cl_2$ as the eluent to afford the title compound in the form of a white glass.

EXAMPLE 76

23-tetrafluorophenylhydrazone-LL-F28249α

In 8 ml of methanol under $N_2$ atmosphere, 80 mg of 23-oxo-LLF28249α is stirred with 80 mg of pentafluorophenylhydrazine and 16 µl of HOAc for 5 hours at room temperature. The mixture was diluted with 200 mL of $CH_2Cl_2$ and washed twice with 200 mL of 5% HCl. The aqueous extracts were combined and reextracted twice with 300 mL of $CH_2Cl_2$. The $CH_2Cl_2$ extracts were combined and washed with 100 mL of saturated $NaHCO_3$, dried ($NaSO_4$), filtered and evaporated. The residue is purified by chromatography on silica gel using 1% isopropanol in $CH_2Cl_2$ as eluent to afford the title compound having a melting point of 142 °C.

EXAMPLE 77

23-oxamic acid hydrazone-LL-F28249α

In 15 mL of refluxing EtoH under $N_2$ atmosphere, 80 mg of 23-oxo-LL-F28249α is stirred with 45 mg oxamic acid hydrazide for 3 days. The mixture is diluted with 200 mL $CH_2Cl_2$ and washed twice with 200 mL 5% HCl. The combined aqueous extract were reextracted twice with 300 mL $CH_2Cl_2$. The combined $CH_2Cl_2$ extracts were combined, washed with 100 mL saturated $NaHCO_3$ dried ($Na_2SO_4$),filtered, and evaporated. The residue is purified by chromatography on silica gel using 2% isopropanol in $CH_2Cl_2$ as eluent to afford the title compound. m.p. 175° (decomp).

**Claims**

**Claims for the following Contracting States : BE, CH, DE, FR, GB, IT, LI, LU, NL, SE**

**1.** The compounds characterized by structural formula (I):

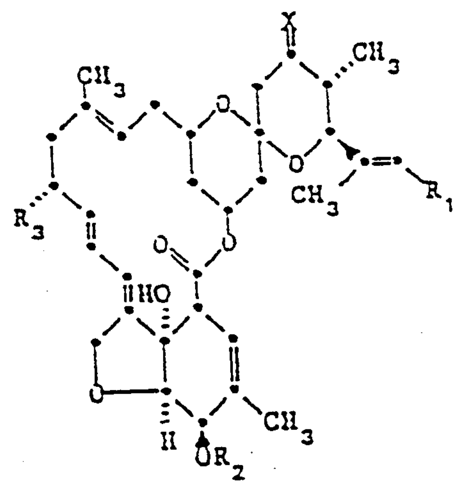

I

wherein $R_1$ is methyl, ethyl or isopropyl; $R_2$ is hydrogen, methyl, $C_1$-$C_4$ alkanoyl, methoxyacetyl, chloroacetyl, dichloroacetyl, trichloroacetyl, trifluoroacetyl, $R_3$ is hydrogen or methyl; X is $NOR_4$, or N-$NHR_5$; $R_4$ is hydrogen, $C_1$-$C_6$ alkyl, benzyl, allyl, propargyl, phenyl, $CH_2COO$-alkyl ($C_1$-$C_4$), N-($C_1$-$C_6$ alkyl)carbamoyl, N-(allyl)carbamoyl, N-(propargyl)carbamoyl, N-(phenyl)carbamoyl, N-(chlorophenyl)-carbamoyl, N-(dichlorophenyl)carbamoyl, N-(benzyl)carbamoyl, $C_1$-$C_6$ alkanoyl, chloroacetyl, methoxyacetyl, phenylacetyl, or chlorobenzoyl; $R_5$ is $C_1$-$C_6$alkanoyl, formyl, $C_1$-$C_6$alkyl, benzoyl, optionally substituted with one or two halogens, $C_1$-$C_4$ alkyl groups, $C_1$-$C_4$ alkoxy groups or nitro groups; and the pharmaceutically and pharmacologically acceptable salts thereof.

**2.** The compound according to Claim 1, wherein $R_1$ is isopropyl; $R_2$ is hydrogen or methyl; $R_3$ is methyl; and X is $NOR_4$; wherein $R_1$ is isopropyl; $R_2$ is hydrogen; $R_3$ is methyl; X is $NOR_4$, N-$NHCH_3$, and $R_4$ is

$C_1$-$C_3$ alkyl, N-($C_1$-$C_6$ alkyl)carbamoyl, N-(allyl)carbamoyl, N-(propargyl)carbamoyl, N-(phenyl)-carbamoyl, N-(4-chlorophenyl)carbamoyl, N-(benzyl)carbamoyl, $C_1$-$C_6$ alkanoyl, chloroacetyl, methoxyacetyl, benzoyl or chlorobenzoyl; wherein $R_1$ is isopropyl; $R_2$ is hydrogen; $R_3$ is methyl; X is $NOR_4$; and $R_4$ is methyl, ethyl, N-($C_1$-$C_4$ alkyl) carbamoyl, N-(phenyl)carbamoyl, or N-(4-chlorophenyl)-carbamoyl; wherein $R_1$ is isopropyl; $R_2$ is hydrogen; $R_3$ is methyl; and X is $NOCH_3$; and wherein $R_1$ is isopropyl; $R_2$ is hydrogen; $R_3$ is methyl; and X is

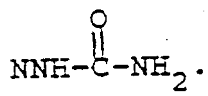

**3.** A method for controlling plant insects topically or systemically, and protecting crops, trees, shrubs, stored grain and ornamentals, said method characterized by: applying an insecticidally-effctive amount of the compound represented by structural formual (I),

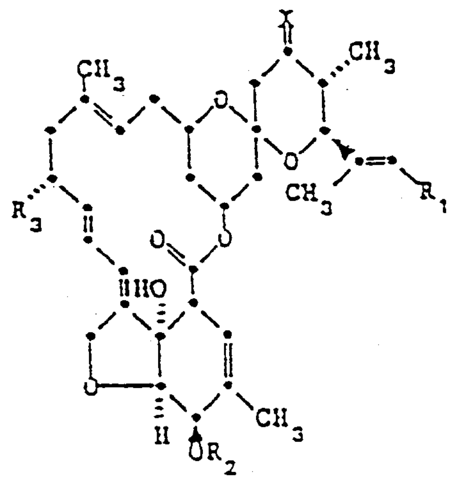

I

wherein $R_1$, $R_2$, $R_3$ and X are as defined in Claim 1.

**4.** A method according to Claim 3 , wherein said compound is $R_1$ as isopropyl; $R_2$ as hydrogen; $R_3$ as methyl and X as $NOCH_3$.

**5.** A method for the control of plant nematodes, said method characterized by: applying to the foilage of plants, the soil in which they are grown or into the trunks thereof, a nematicidally-effective amount of the compound represented by structural formula (I),

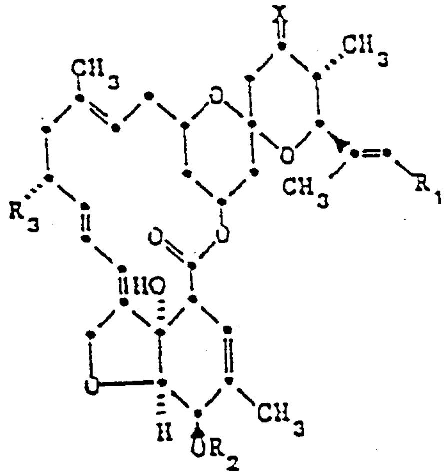

I

wherein $R_1$, $R_2$, $R_3$ and X are as defined in Claim 1.

**6.** A method according to claim 5, wherein said compound is $R_1$ as isopropyl; $R_2$ as hydrogen; $R_3$ as methyl; and X $NOCH_3$.

**7.** A composition for the treatment, prevention or control of endo- and/or ectoparasitic infections in warm-blooded animals, said composition characterized by: a prophylactically, therapeutically or pharmaceutically-effective amount of the compound represented by structural formual (I),

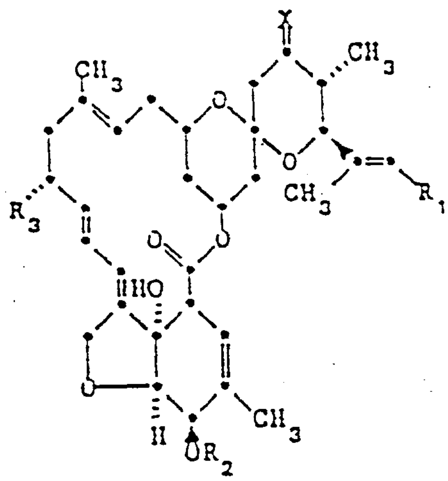

I

wherein $R_1$, $R_2$, $R_3$ and X are as defined in Claim 1.

**8.** A composition for controlling insects, said composition characterized by: an insecticidally-effective amount of the compound represented by the structural formula (I),

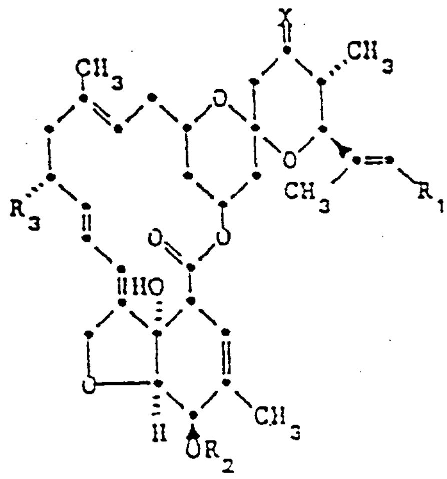

I

wherein $R_1$, $R_2$, $R_3$ and X are as defined in Claim 1.

**9.** A compound characterized by:
23-(formylhydrazone)-LL-F28249α;
23-[(tolylsulfonyl)hydrazone]-LL-F28249α;
23-(4-phenylsemicarbazone)-LL-F28249α;
23-[(2,3,4,5,6-pentafluorophenyl)hydrazone]-LL-F28249α;
23-oxamoylhydrazone-LL-F28249α;
23-[o-(phenoxyacetoxy)oxime]-LL-F28249α;
23-[o-(ethoxymethyl)oxime]-LL-F28249α;
23-o-methyloxime-5-(phenoxyacetoxy)-LL-F28249α;
23-(4-phenyl-3-thiosemicarbazone)-LL-F28249α;
5-O-t-butyldimethylsilyl-23-oxime-LL-F28249α; and
5-O-Trimethylsilyl-23-oxime-LL-F28249α;
or the β, γ, δ, ε, ζ, θ, ι and λ derivatives of each of the above said silyl compounds.

**10.** A method for preparing a compound of Claim 1 which comprises the preparation of a 5-O-tri-substituted silyl-LL-F28249α, β, γ, δ, ε, ζ, θ, ι or λor a 5-O-trisubstituted silyloxyacetyl LB-F28249α, β, γ, δ, ε, ζ, θ, ι or λ having the structure,

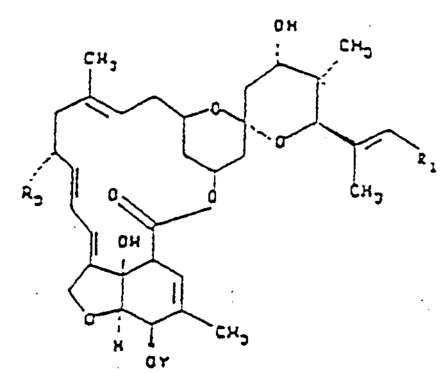

wherein $R_1$ is methyl, ethyl or isopropyl; $R_3$ is hydrogen or methyl; Y is a trisubstituted silyl or 5-trisubstituted silyloxyacetyl function such as t-butyldimethylsilyl, trimethylsilyl or tbutyldimethylsilyloxyacetyl; characterized by: reacting a compound having the structure

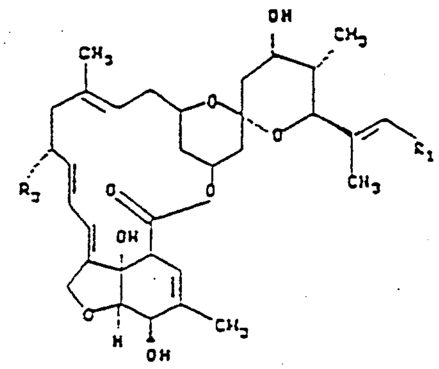

wherein $R_1$ and $R_3$ are as described above, with a trisubstituted silyl halide or a trisubstituted silyloxyacetyl halide, in the presence of an organic base and an aprotic solvent, at a temperature of between about 0 ° C and 30 ° C, whereby the desired 5-O-trisubstituted silyl-LL-F28249α, β, γ, δ, ε, ζ, θ, ι or λ, or 5-O-trisubstituted silyloxyacetyl-LL-F28249α, β, γ, δ, ε, ζ, θ, ι or λ compound is obtained.

**11.** A method according to Claim 10, wherein said organic base is imidazole, pyridine, 4-dimethylaminopyridine or triethylamine; the aprotic solvent is methylene chloride, toluene, ethylacetate, tetrahydrofuran or ethylenechloride and the trisubstituted silyl function is t-butyldimethylsilyl, trimethylsilyl or t-butyldimethylsilyloxyacetyl.

**12.** A method for preparing a compound of Claim 1 which comprises the preparation of a 5-O-trisubstituted silyl-23-oxo-LL-F28249α, β, γ, δ, ε, ζ, θ, ι and λ having the structure,

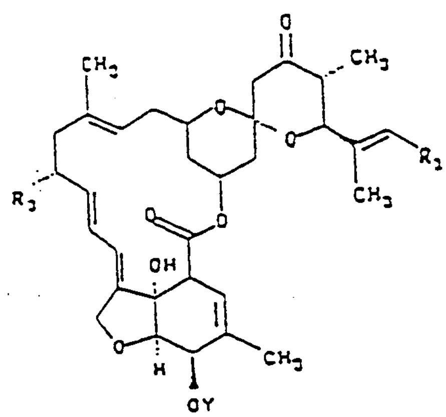

wherein $R_1$ is methyl, ethyl or isopropyl; $R_3$ is hydrogen or methyl and Y is a trisubstituted silyl or trisubstituted silyloxyacetyl function such as t-butyldimethylsilyl, trimethylsilyl or t-butyldimethylsilyloxyacetyl; characterized by: reacting a compound having the structure,

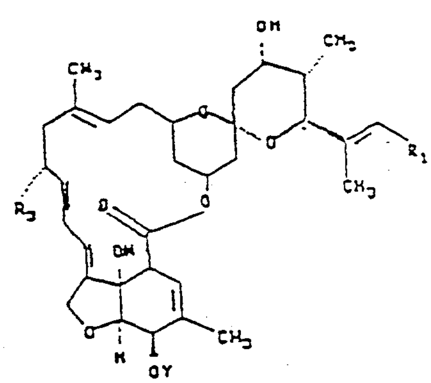

wherein $R_1$, $R_3$ and Y are as described above, with an oxidizing agent such as pyridinium dichromate, pyridinium chlorochromate, chronic acid-dimethylpyrazole, acetic anhydride/dimethylsulfoxide, N-chlorosuccinimide/dimethylsulfoxide, oxalyl or chloride/dimethylsulfoxide; in the presence of an aprotic solvent and optionally an organic base, at a temperature between -78 °C and +25 °C.

**13.** A method according to Claim 12, wherein the organic base is triethylamine or diisopropylethylamine; the aprotic solvent is methylene chloride, ethylenechloride, dimethylformamide or dimethylsulfoxide; whereby the desired 5-O-trisubstituted silyl-23-oxo-LL-F28249α,β, γ, δ, ε, ζ, θ, ι or λ or 5-O-trisubstituted silyloxyacetyl-23-oxo-LL-F28249α, β, γ, δ, ε, ζ, θ, ι or λ compound, is obtained.

**14.** A compound characterized by:
5-O-t-butyldimethylsilyl-LL-F28249α;
5-O-Trimethylsilyl-LL-F28249α;
5-O-t-butyldimethysilyl-23-oxo-LL-F28249α;
5-O-t-trimethysilyl-23-oxo-LL-F28249α;
and
5-O-t-butyldimethysilyloxyacetyl-LL-F28249α or the β, γ, δ, ε, ζ, θ, ι and λ derivatives of each of the above said silyl compounds.

## Claims for the following Contracting States : AT, ES, GR

**1.** A method for preparing a compound characterized by structural formula (I):

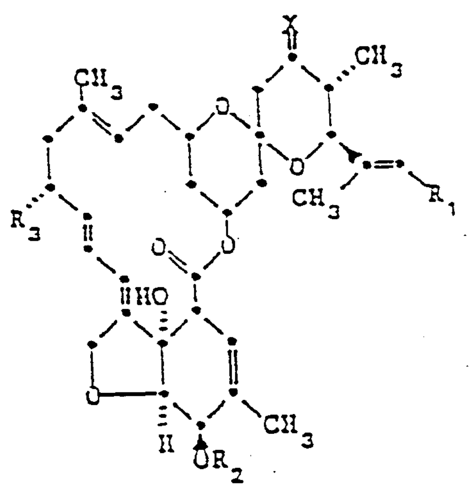

I

wherein $R_1$ is methyl, ethyl or isopropyl; $R_2$ is hydrogen, methyl, $C_1$-$C_4$ alkanoyl, methoxyacetyl, chloroacetyl, dichloroacetyl, trichloroacetyl, trifluoroacetyl, $R_3$ is hydrogen or methyl; X is $NOR_4$, or N-$NHR_5$; $R_4$ is hydrogen, $C_1$-$C_6$ alkyl, benzyl, allyl, propargyl, phenyl, $CH_2COO$-alkyl ($C_1$-$C_4$), N-($C_1$-$C_6$ alkyl)carbamoyl, N-(allyl)carbamoyl, N-(propargyl)carbamoyl, N-(phenyl)carbamoyl, N-(chlorophenyl)-carbamoyl, N-(dichlorophenyl)carbamoyl, N-(benzyl)carbamoyl, $C_1$-$C_6$ alkanoyl, chloroacetyl, methoxyacetyl, phenylacetyl, or chlorobenzoyl; $R_5$ is $C_1$-$C_6$alkanoyl, formyl, $C_1$-$C_6$alkyl, benzoyl, optionally substituted with one or two halogens, $C_1$-$C_4$ alkyl groups, $C_1$-$C_4$ alkoxy groups or nitro groups; and the pharmaceutically and pharmacologically acceptable salts thereof,

which comprises the preparation of a 5-O-trisubstituted silyl-LL-F28249α, β, γ, δ, ε, ζ, θ, ι or λ or a 5-O-trisubstituted silyloxyacetyl LL-F28249α, β, γ, δ, ε, ζ, θ, ι or λ having the structure,

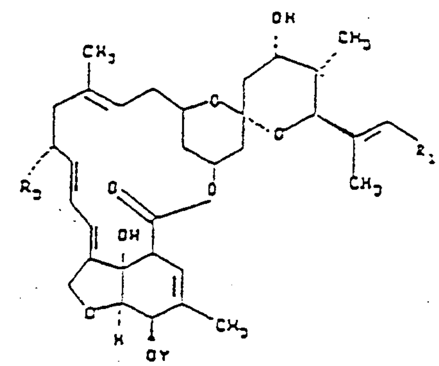

wherein $R_1$ is methyl, ethyl or isopropyl; $R_3$ is hydrogen or methyl; Y is a trisubstituted silyl or 5-trisubstituted silyloxyacetyl function such as t-butyldimethylsilyl, trimethysilyl or tbutyldimethylsilyloxyacetyl; characterized by: reacting a compound having the structure

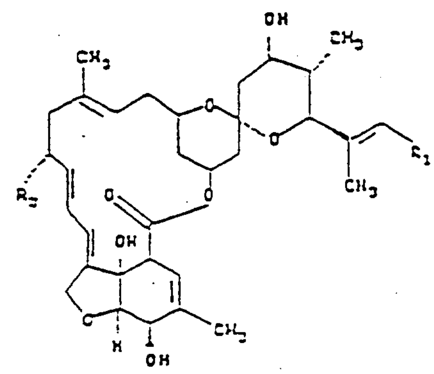

wherein $R_1$ and $R_3$ are as described above, with a trisubstituted silyl halide or a trisubstituted silyloxyacetyl halide, in the presence of an organic base and an aprotic solvent, at a temperature of between about 0 °C and 30 °C, whereby the desired 5-O-trisubstituted silyl-LL-F28249α, β, γ, δ, ε, ζ, θ, ι or λ, or 5-O-trisubstituted silyloxyacetyl-LL-F28249α, β, γ, δ, ε, ζ, θ, ι or λ compound is obtained.

**2.** A method according to Claim 1, wherein said organic base is imidazole, pyridine, 4-dimethylaminopyridine or triethylamine; the aprotic solvent is methylene chloride, toluene, ethylacetate tetrahydrofuran or ethylenechloride and the trisubstituted silyl function is t-butyldimethysilyl, trimethylsilyl or t-butyldimethylsilyloxyacetyl.

**3.** A method for preparing a compound of Claim 1 which comprises the preparation of a 5-O-trisubstituted silyl-23-oxo-LL-F28249α, β, γ, δ, ε, ζ, θ, ι and λ having the structure,

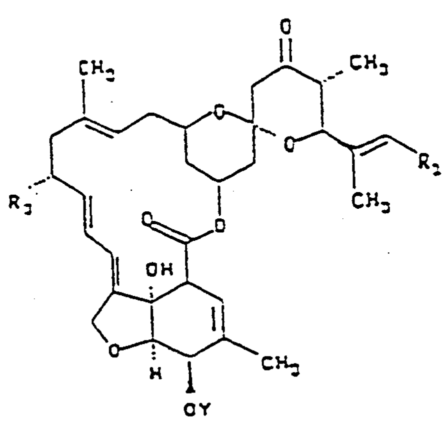

wherein $R_1$ is methyl, ethyl or isopropyl; $R_3$ is hydrogen or methyl and Y is a trisubstituted silyl or trisubstituted silyloxyacetyl function such as t-butyldimethylsilyl, trimethysilyl or t-butyldimethylsilyloxyacetyl; characterized by: reacting a compound having the structure,

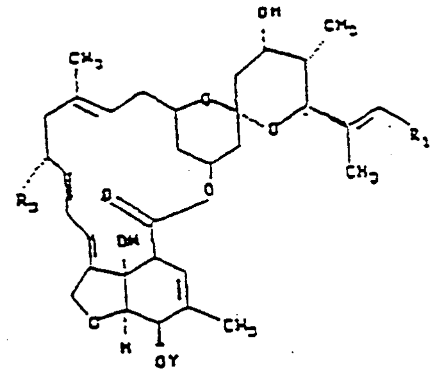

wherein $R_1$, $R_3$ and Y are as described above, with an oxidizing agent such as pyridinium dichromate, pyridinium chlorochromate, chronic acid-dimethylpyrazole, acetic anhydride/dimethylsulfoxide, N-chlorosuccinimide/dimethylsulfoxide, oxalyl or chloride/dimethylsulfoxide; in the presence of an aprotic solvent and optionally an organic base, at a temperature between -78 °C and +25 °C.

**4.** A method according to Claim 3 wherein the organic base is triethylamine or diisopropylethylamine; the aprotic solvent is methylene chloride, ethylenechloride, dimethylformamide or dimethylsulfoxide; whereby the desired 5-O-trisubstituted silyl-23-oxo-LL-F28249$\alpha$,$\beta$, $\gamma$, $\delta$, $\epsilon$, $\zeta$, $\theta$, $\iota$ or $\lambda$ or 5-O-trisubstituted silyloxyacetyl-23-oxo-LL-F28249$\alpha$, $\beta$, $\gamma$, $\delta$, $\epsilon$, $\zeta$, $\theta$, $\iota$ or $\lambda$ compound, is obtained.

**5.** The method according to Claim 1, wherein $R_1$ is isopropyl; $R_2$ is hydrogen or methyl; $R_3$ is methyl; and X is $NOR_4$; wherein $R_1$ is isopropyl; $R_2$ is hydrogen; $R_3$ is methyl; X is $NOR_4$, N-$NHCH_3$, and $R_4$ is $C_1$-$C_3$ alkyl, N-($C_1$-$C_6$ alkyl)carbamoyl, N-(allyl)carbamoyl, N-(propargyl)carbamoyl, N-(phenyl)carbamoyl, N-(4-chlorophenyl)carbamoyl, N-(benzyl)carbamoyl, $C_1$-$C_6$ alkanoyl, chloroacetyl, methoxyacetyl, benzoyl or chlorobenzoyl; wherein $R_1$ is isopropyl; $R_2$ is hydrogen; $R_3$ is methyl; X is $NOR_4$; and $R_4$ is methyl, ethyl, N-($C_1$-$C_4$ alkyl) carbamoyl, N-(phenyl)carbamoyl, or N-(4-chlorophenyl)carbamoyl; wherein $R_1$ is isopropyl; $R_2$ is hydrogen; $R_3$ is methyl; and X is $NOCH_3$; and wherein $R_1$ is isopropyl; $R_2$ is hydrogen; $R_3$ is methyl; and X is

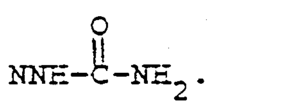

**6.** The method according to Claim 1 which produces
23-(formylhydrazone)-LL-F28249$\alpha$;
23-[(tolylsulfonyl)hydrazone]-LL-F28249$\alpha$;
23-(4-phenylsemicarbazone)-LL-F28249$\alpha$;
23-[(2,3,4,5,6-pentafluorophenyl)hydrazone]-LL-F28249$\alpha$;
23-oxamoylhydrazone-LL-F28249$\alpha$;
23-[o-(phenoxyacetoxy)oxime]-LL-F28249$\alpha$;
23-[o-(ethoxymethyl)oxime]-LL-F28249$\alpha$;
23-o-methyloxime-5-(phenoxyacetoxy)-LL-F28249$\alpha$;
23-(4-phenyl-3-thiosemicarbazone)-LL-F28249$\alpha$;
5-O-t-butyldimethylsilyl-23-oxime-LL-F28249$\alpha$;
5-O-Trimethylsilyl-23-oxime-LL-F28249$\alpha$; or the $\beta$, $\gamma$, $\delta$, $\epsilon$, $\zeta$, $\theta$, $\iota$ and $\lambda$ derivatives of each of the above said silyl compounds.

7. A method for controlling plant insects topically or systemically, and protecting crops, trees, shrubs, stored grain and ornamentals, said method characterized by: applying an insecticidally-effective amount of the compound represented by structural formual (I),

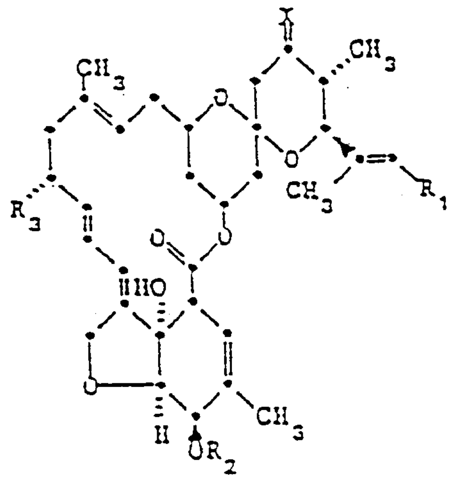

I

wherein $R_1$, $R_2$, $R_3$ and X are as defined in Claim 1.

8. A method according to Claim 7, wherein said compound is $R_1$ as isopropyl; $R_2$ as hydrogen; $R_3$ as methyl and X as $NOCH_3$.

9. A method for the control of plant nematodes, said method characterized by: applying to the foilage of plants, the soil in which they are grown or into the trunks thereof, a nematicidally-effective amount of the compound represented by structural formula (I),

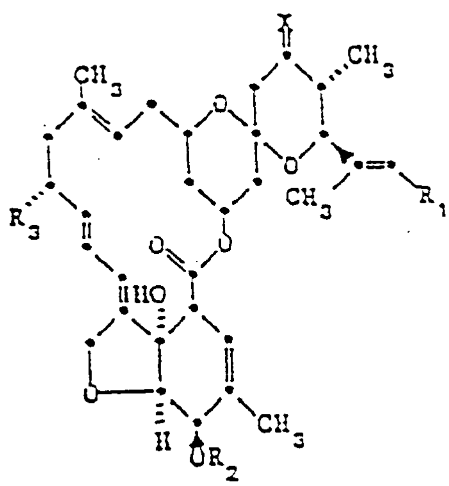

I

wherein $R_1$, $R_2$, $R_3$ and X are as defined in Claim 1.

10. A method according to Claim 9, wherein said compound is $R_1$ as isopropyl; $R_2$ as hydrogen; $R_3$ as methyl; and X $NOCH_3$.

## Patentansprüche

**Patentansprüche für folgende Vertragsstaaten : BE, CH, DE, FR, GB, IT, LI, LU, NL, SE**

1. Verbindungen, gekennzeichnet durch die Strukturformel (I):

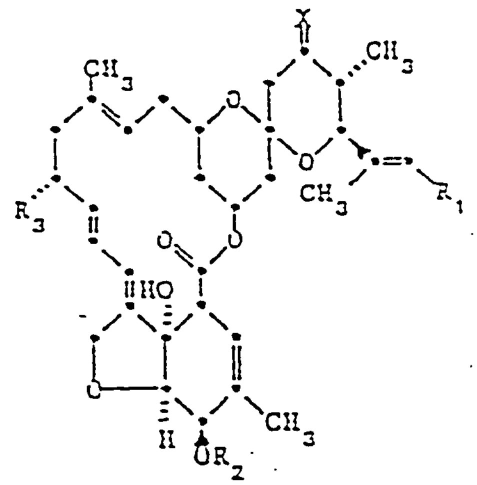

I

in der $R_1$ Methyl, Ethyl oder Isopropyl ist; $R_2$ Wasserstoff, Methyl, $C_1$-$C_4$-Alkanoyl, Methoxyacetyl, Chloracetyl, Dichloracetyl, Trichloracetyl, Trifluoracetyl ist, $R_3$ Wasserstoff oder Methyl ist; X $NOR_4$ oder $N$-$NHR_5$ ist; $R_4$ Wasserstoff, $C_1$-$C_6$-Alkyl, Benzyl, Allyl, Propargyl, Phenyl, $CH_2COO$-$C_1$-$C_4$-alkyl, N-($C_1$-$C_6$-Alkyl)carbamoyl, N-(Allyl)carbamoyl, N-(Propargyl)carbamoyl, N-(Phenyl)carbamoyl, N-(Chlorphenyl)carbamoyl, N-(Dichlorphenyl)carbamoyl, N-(Benzyl)carbamoyl, $C_1$-$C_6$-Alkanoyl, Chloracetyl, Methoxyacetyl, Phenylacetyl oder Chlorbenzoyl ist; $R_5$ $C_1$-$C_6$-Alkanoyl, Formyl, $C_1$-$C_6$-Alkyl, Benzoyl, gegebenenfalls mit einem bzw. einer oder zwei Halogenen, $C_1$-$C_4$-Alkylgruppen, $C_1$-$C_4$-Alkoxygruppen oder Nitrogruppen substituiert, ist; und ihre pharmazeutisch und pharmakologisch annehmbaren Salze.

2. Verbindung nach Anspruch 1, worin $R_1$ Isopropyl ist; $R_2$ Wasserstoff oder Methyl ist; $R_3$ Methyl ist; und X $NOR_4$ ist; worin $R_1$ Isopropyl ist; $R_2$ Wasserstoff ist; $R_3$ Methyl ist; X $NOR_4$, $N$-$NHCH_3$ ist und $R_4$ $C_1$-$C_3$-Alkyl, N-($C_1$-$C_6$-Alkyl)carbamoyl, N-(Allyl)carbamoyl, N-(Propargyl)carbamoyl, N-(Phenyl)carbamoyl, N-(4-Chlorphenyl)carbamoyl, N-(Benzyl)carbamoyl, $C_1$-$C_6$-Alkanoyl, Chloracetyl, Methoxyacetyl, Benzoyl oder Chlorbenzoyl ist; worin $R_1$ Isopropyl ist; $R_2$ Wasserstoff ist; $R_3$ Methyl ist; X $NOR_4$ ist; und $R_4$ Methyl, Ethyl, N-($C_1$-$C_4$-Alkyl)carbamoyl, N-(Phenyl)carbamoyl oder N-(4-Chlorphenyl)carbamoyl ist; worin $R_1$ Isopropyl ist; $R_2$ Wasserstoff ist; $R_3$ Methyl ist; und X $NOCH_3$ ist; und worin $R_1$ Isopropyl ist; $R_2$ Wasserstoff ist; $R_3$ Methyl ist; und X

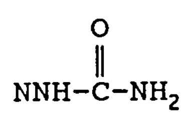

ist.

3. Verfahren zur topischen oder systemischen Kontrolle von Pflanzeninsekten und zum Schutz von Feldfrüchten, Bäumen, Sträuchern, gelagertem Korn und Zierpflanzen, wobei das Verfahren gekennzeichnet ist durch:
Anwenden einer insektizid wirksamen Menge der Verbindung, die durch die Strukturformel (I) dargestellt wird,

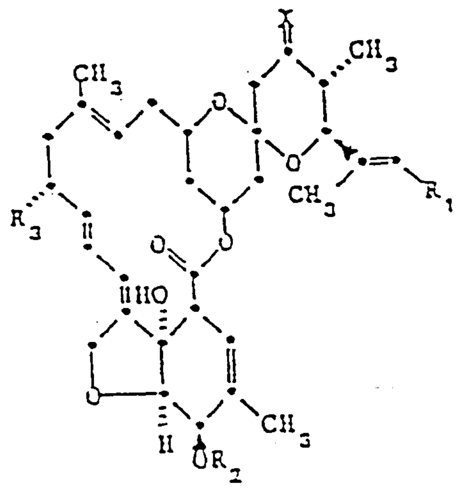

I

in der $R_1$, $R_2$, $R_3$ und X wie in Anspruch 1 definiert sind.

4. Verfahren nach Anspruch 3, worin die Verbindung mit $R_1$ als Isopropyl; $R_2$ als Wasserstoff; $R_3$ als Methyl und X als $NOCH_3$ vorliegt.

5. Verfahren zur Kontrolle von Pflanzennematoden, wobei das Verfahren gekennzeichnet ist durch:
Auftragen einer nematizid wirksamen Menge der Verbindung, die durch die Strukturformel (I) dargestellt wird,

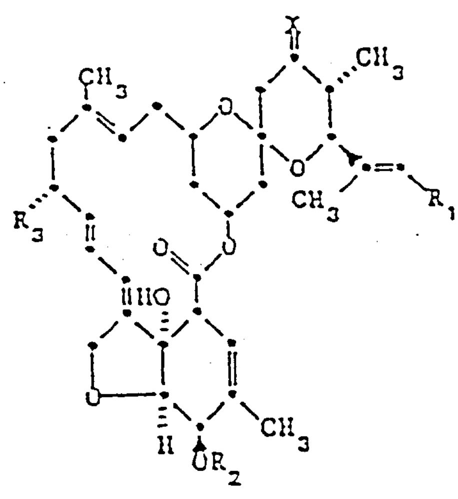

I

in der $R_1$, $R_2$, $R_3$ und X wie in Anspruch 1 definiert sind, auf die Blätter von Pflanzen, den Boden, in dem sie wachsen, oder in deren Stämme.

6. Verfahren nach Anspruch 5, in dem die Verbindung mit $R_1$ als Isopropyl; $R_2$ als Wasserstoff; $R_3$ als Methyl; und X als $NOCH_3$ vorliegt.

7. Zusammensetzung zur Behandlung, Verhütung oder Kontrolle von Endo- und/oder Ektoparasiten-Infektionen bei warmblütigen Tieren, wobei die Zusammensetzung gekennzeichnet ist durch:
eine prophylaktisch, therapeutisch oder pharmazeutisch wirksame Menge der Verbindung, die durch die Strukturformel (I) dargestellt wird,

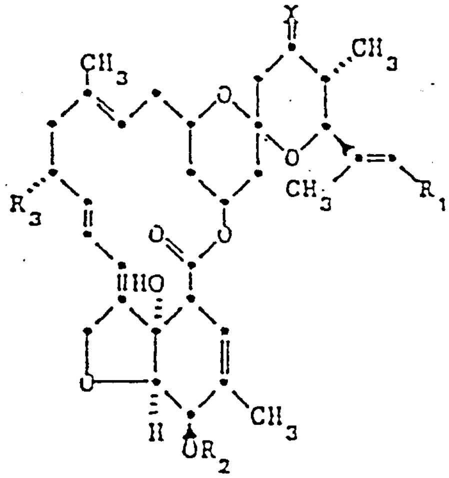

I

in der $R_1$, $R_2$, $R_3$ und X wie in Anspruch 1 definiert sind.

**8.** Zusammensetzung zur Kontrolle von Insekten, wobei die Zusammensetzung gekennzeichnet ist durch: eine insektizid wirksame Menge der Verbindung, die durch die Strukturformel (I) dargestellt wird,

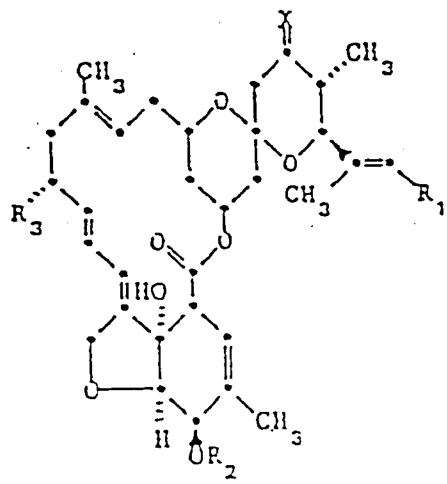

I

in der $R_1$, $R_2$, $R_3$ und X wie in Anspruch 1 definiert sind.

**9.** Verbindung, gekennzeichnet durch:
23-(Formylhydrazon)-LL-F28249α;
23-[(Tolylsulfonyl)hydrazon]-LL-F28249α;
23-(4-Phenylsemicarbazon)-LL-F28249α;
23-[(2,3,4,5,6-Pentafluorphenyl)hydrazon]-LL-F28249α;
23-Oxamoylhydrazon-LL-F28249α;
23-[o-(Phenoxyacetoxy)oxim]-LL-F28249α;
23-[o-(Ethoxymethyl)oxim]-LL-F28249α;
23-o-Methyloxim-5-(phenoxyacetoxy)-LL-F28249α;
23-(4-Phenyl-3-thiosemicarbazon)-LL-F28249α;
5-O-t-Butyldimethylsilyl-23-oxim-LL-F28249α; und
5-O-Trimethylsilyl-23-oxim-LL-F28249α;
oder die β-, γ-, δ-, ε-, ζ-, θ-, ι- und λ-Derivate jeder der obigen Silyl-Verbindungen.

**10.** Verfahren zur Herstellung einer Verbindung nach Anspruch 1, umfassend die Herstellung eines 5-O-trisubstituierten Silyl-LL-F28249α, β, γ, δ, ε, ζ, θ, ι oder λ oder oder eines 5-O-trisubstituierten Silyloxyacetyl-LL-F28249α, β, γ, δ, ε, ζ, θ, ι oder λ mit der Struktur

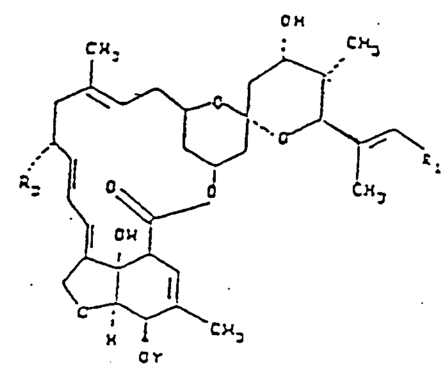

in der $R_1$ Methyl, Ethyl oder Isopropyl ist; $R_3$ Wasserstoff oder Methyl ist; Y eine trisubstituierte Silyl- oder 5-trisubstituierte Silyloxyacetyl-Funktion, wie z.B. t-Butyldimethylsilyl, Trimethylsilyl oder t-Butyldimethylsilyloxyacetyl, ist; gekennzeichnet durch:
Umsetzen einer Verbindung mit der Struktur

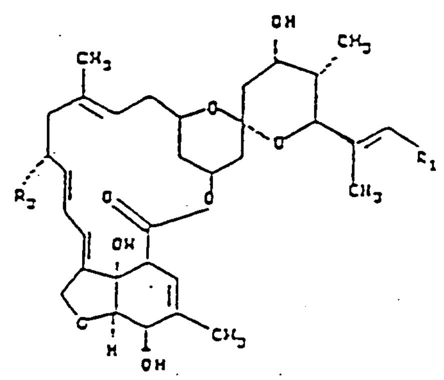

in der $R_1$ und $R_3$ wie oben beschrieben sind, mit einem trisubstituierten Silylhalogenid oder einem trisubstituierten Silyloxyacetylhalogenid in Anwesenheit einer organischen Base und eines aprotischen Lösungsmittels bei einer Temperatur zwischen ungefähr 0 °C und 30 °C, wodurch die gewünschte 5-O-trisubstituierte Silyl-LL-F28249α-, β-, γ-, δ-, ε-, ζ-, θ-, ι- oder λ- oder 5-O-trisubstituierte Silyloxyacetyl-LL-F28249α-, β-, γ-, δ-, ε-, ζ-, θ-, ι- oder λ- Verbindung erhalten wird.

**11.** Verfahren nach Anspruch 10, in dem die organische Base Imidazol, Pyridin, 4-Dimethylaminopyridin oder Triethylamin ist; das aprotische Lösungsmittel Methylenchlorid, Toluol, Ethylacetat, Tetrahydrofuran oder Ethylenchlorid ist und die trisubstituierte Silyl-Funktion t-Butyldimethylsilyl, Trimethylsilyl oder t-Butyldimethylsilyl oxyacetyl ist.

**12.** Verfahren zur Herstellung einer Verbindung nach Anspruch 1, umfassend die Herstellung eines 5-O-trisubstituierten Silyl-23-oxo-LL-F28249α, β, γ, δ, ε, ζ, θ, ι und λ mit der Struktur

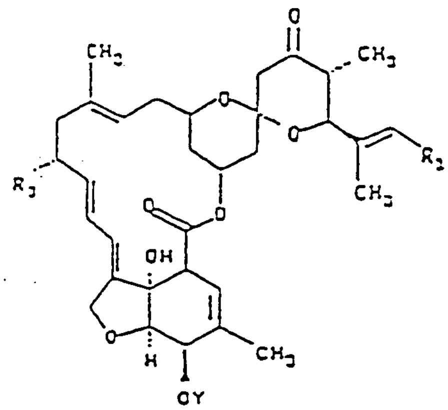

worin $R_1$ Methyl, Ethyl oder Isopropyl ist; $R_3$ Wasserstoff oder Methyl ist und Y eine trisubstituierte Silyl- oder trisubstituierte Silyloxyacetyl-Funktion, wie beispielsweise t-Butyldimethylsilyl, Trimethylsilyl oder t-Butyldimethylsilyloxyacetyl, ist; gekennzeichnet durch:
Umsetzen einer Verbindung mit der Struktur

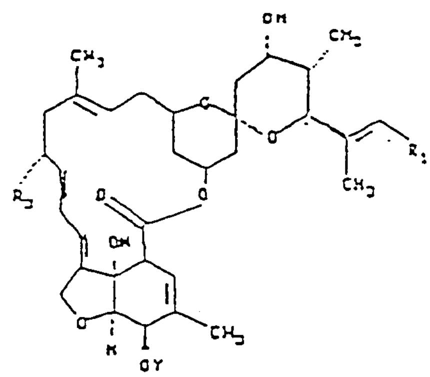

worin $R_1$, $R_3$ und Y wie oben beschrieben sind, mit einem Oxidationsmittel, wie z.B. Pyridiniumdichromat, Pyridiniumchlorochromat, Chromsäure-Dimethylpyrazol, Essigsäureanhydrid/Dimethylsulfoxid, N-Chlorsuccinimid/Dimethylsulfoxid, Oxalylchlorid/Dimethylsulfoxid; in Gegenwart eines aprotischen Lösungsmittels und gegebenenfalls einer organischen Base bei einer Temperatur zwischen -78 °C und +25 °C.

**13.** Verfahren nach Anspruch 12, in dem die organische Base Triethylamin oder Diisopropylethylamin ist; das aprotische Lösungsmittel Methylenchlorid, Ethylenchlorid, Dimethylformamid oder Dimethylsulfoxid ist; wodurch die gewünschte 5-O-trisubstituierte Silyl-23-oxo-LL-F28249$\alpha$-, $\beta$-, $\gamma$-, $\delta$-, $\epsilon$-, $\zeta$-, $\theta$-, $\iota$- oder $\lambda$- oder 5-O-trisubstituierte Silyloxyacetyl-23-oxo-LL-F28249$\alpha$-, $\beta$-, $\gamma$-, $\delta$-, $\epsilon$-, $\zeta$-, $\theta$-, $\iota$- oder $\lambda$-Verbindung erhalten wird.

**14.** Verbindung, gekennzeichnet durch:
5-O-t-Butyldimethylsilyl-LL-F28249$\alpha$;
5-O-Trimethylsilyl-LL-F28249$\alpha$;
5-O-t-Butyldimethylsilyl-23-oxo-LL-F28249$\alpha$;
5-O-Trimethylsilyl-23-oxo-LL-F28249$\alpha$; und
5-O-t-Butyldimethylsilyloxyacetyl-LL-F28249$\alpha$ oder die $\beta$-, $\gamma$-, $\delta$-, $\epsilon$-, $\zeta$-, $\theta$-, $\iota$- und $\lambda$-Derivate jeder der obigen Silyl-Verbindungen.

**Patentansprüche für folgende Vetragsstaaten : AT, ES, GR**

1. Verfahren zur Herstellung einer Verbindung, gekennzeichnet durch die Strukturformel (I):

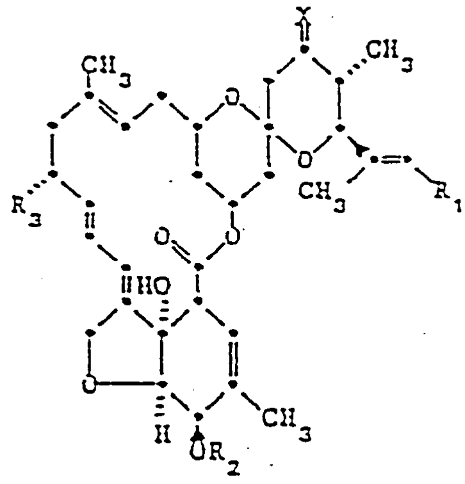

I

in der $R_1$ Methyl, Ethyl oder Isopropyl ist; $R_2$ Wasserstoff, Methyl, $C_1$-$C_4$-Alkanoyl, Methoxyacetyl, Chloracetyl, Dichloracetyl, Trichloracetyl, Trifluoracetyl ist, $R_3$ Wasserstoff oder Methyl ist; X $NOR_4$ oder N-$NHR_5$ ist; $R_4$ Wasserstoff, $C_1$-$C_6$-Alkyl, Benzyl, Allyl, Propargyl, Phenyl, $CH_2COO$-$C_1$-$C_4$-alkyl, N-($C_1$-$C_6$-Alkyl)carbamoyl, N-(Allyl)carbamoyl, N-(Propargyl)carbamoyl, N-(Phenyl)carbamoyl, N-(Chlorphenyl)carbamoyl, N-(Dichlorphenyl)carbamoyl, N-(Benzyl)carbamoyl, $C_1$-$C_6$-Alkanoyl, Chloracetyl, Methoxyacetyl, Phenylacetyl oder Chlorbenzoyl ist; $R_5$ $C_1$-$C_6$-Alkanoyl, Formyl, $C_1$-$C_6$-Alkyl, Benzoyl, gegebenenfalls mit einem bzw. einer oder zwei Halogenen, $C_1$-$C_4$-Alkylgruppen, $C_1$-$C_4$-Alkoxygruppen oder Nitrogruppen substituiert, ist; und deren pharmazeutisch und pharmakologisch annehmbaren Salzen,
umfassend die Herstellung eines 5-O-trisubstituierten Silyl-LL-F28249$\alpha$, $\beta$, $\gamma$, $\delta$, $\epsilon$, $\zeta$, $\theta$, $\iota$ oder $\lambda$ oder eines 5-O-trisubstituierten Silyloxyacetyl-LL-F28249$\alpha$, $\beta$, $\gamma$, $\delta$, $\epsilon$, $\zeta$, $\theta$, $\iota$ oder $\lambda$ mit der Struktur

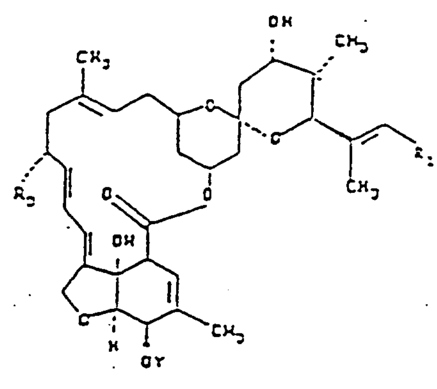

in der $R_1$ Methyl, Ethyl oder Isopropyl ist; $R_3$ Wasserstoff oder Methyl ist; Y eine trisubstituierte Silyl- oder 5-trisubstituierte Silyloxyacetyl-Funktion, wie z.B. t-Butyldimethylsilyl, Trimethylsilyl oder t-Butyldimethylsilyloxyacetyl, ist; gekennzeichnet durch:
Umsetzen einer Verbindung mit der Struktur

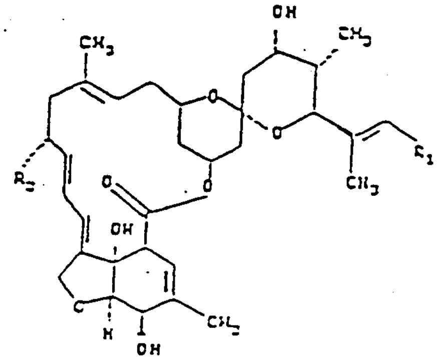

in der $R_1$ und $R_3$ wie oben beschrieben sind, mit einem trisubstituierten Silylhalogenid oder einem trisubstituierten Silyloxyacetylhalogenid in Anwesenheit einer organischen Base und eines aprotischen Lösungsmittels bei einer Temperatur zwischen ungefähr 0 °C und 30 °C, wodurch die gewünschte 5-O-trisubstituierte Silyl-LL-F28249α-, β-, γ-, δ-, ε-, ζ-, θ-, ι- oder λ- oder 5-O-trisubstituierte Silyloxyacetyl-LL-F28249α-, β-, γ-, δ-, ε-, ζ-, θ-, ι- oder λ- Verbindung erhalten wird.

**2.** Verfahren nach Anspruch 1, in dem die organische Base Imidazol, Pyridin, 4-Dimethylaminopyridin oder Triethylamin ist; das aprotische Lösungsmittel Methylenchlorid, Toluol, Ethylacetat, Tetrahydrofuran oder Ethylenchlorid ist und die trisubstituierte Silyl-Funktion t-Butyldimethylsilyl, Trimethylsilyl oder t-Butyldimethylsilyloxyacetyl.

**3.** Verfahren zur Herstellung einer Verbindung nach Anspruch 1, umfassend die Herstellung eines 5-O-trisubstituierten Silyl-23-oxo-LL-F28249α, β, γ, δ, ε, ζ, θ, ι und λ und mit der Struktur

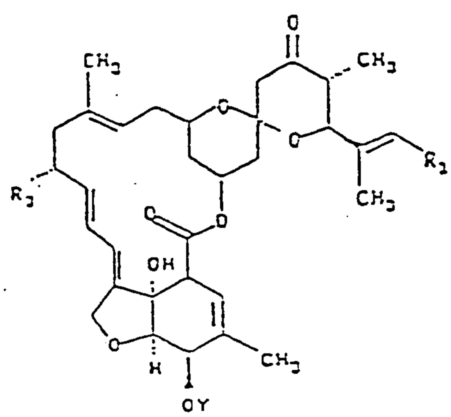

worin $R_1$ Methyl, Ethyl oder Isopropyl ist; $R_3$ Wasserstoff oder Methyl ist und Y eine trisubstituierte Silyl- oder trisubstituierte Silyloxyacetyl-Funktion, wie beispielsweise t-Butyldimethylsilyl, Trimethylsilyl oder t-Butyldimethylsilyloxyacetyl, ist; gekennzeichnet durch:
Umsetzen einer Verbindung mit der Struktur

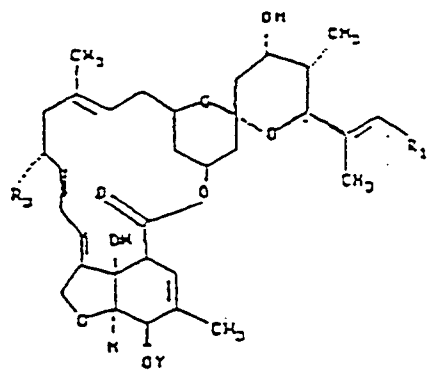

worin $R_1$, $R_3$ und Y wie oben beschrieben sind, mit einem Oxidationsmittel, wie z.B. Pyridiniumdichromat, Pyridiniumchlorochromat, Chromsäure-Dimethylpyrazol, Essigsäureanhydrid/Dimethylsulfoxid, N-Chlorsuccinimid/Dimethylsulfoxid, Oxalylchlorid/Dimethylsulfoxid; in Gegenwart eines aprotischen Lösungsmittels und gegebenenfalls einer organischen Base bei einer Temperatur zwischen -78 °C und +25 °C.

4. Verfahren nach Anspruch 3, in dem die organische Base Triethylamin oder Diisopropylethylamin ist; das aprotische Lösungsmittel Methylenchlorid, Ethylenchlorid, Dimethylformamid oder Dimethylsulfoxid ist; wodurch die gewünschte 5-O-trisubstituierte Silyl-23-oxo-LL-F28249α-, β-, γ-, δ-, ε-, ζ-, θ-, ι- oder λ- oder 5-O-trisubstituierte Silyloxyacetyl-23-oxo-LL-F28249α-, β-, γ-, δ-, ε-, ζ-, θ-, ι- oder λ-Verbindung erhalten wird.

5. Verfahren nach Anspruch 1, worin $R_1$ Isopropyl ist; $R_2$ Wasserstoff oder Methyl ist; $R_3$ Methyl ist; und X $NOR_4$ ist; worin $R_1$ Isopropyl ist; $R_2$ Wasserstoff ist; $R_3$ Methyl ist; X $NOR_4$, $N\text{-}NHCH_3$ ist und $R_4$ $C_1$-$C_3$-Alkyl, N-($C_1$-$C_6$-Alkyl)carbamoyl, N-(Allyl)carbamoyl, N-(Propargyl)carbamoyl, N-(Phenyl)carbamoyl, N-(4-Chlorphenyl)carbamoyl, N-(Benzyl)carbamoyl, $C_1$-$C_6$-Alkanoyl, Chloracetyl, Methoxyacetyl, Benzoyl oder Chlorbenzoyl ist; worin $R_1$ Isopropyl ist; $R_2$ Wasserstoff ist; $R_3$ Methyl ist; X $NOR_4$ ist; und $R_4$ Methyl, Ethyl, N-($C_1$-$C_4$-Alkyl)carbamoyl, N-(Phenyl)carbamoyl oder N-(4-Chlorphenyl)carbamoyl ist; worin $R_1$ Isopropyl ist; $R_2$ Wasserstoff ist; $R_3$ Methyl ist; und X $NOCH_3$ ist; und worin $R_1$ Isopropyl ist; $R_2$ Wasserstoff ist; $R_3$ Methyl ist; und X

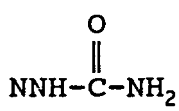

ist.

6. Verfahren nach Anspruch 1, welches erzeugt:
23-(Formylhydrazon)-LL-F28249α;
23-[(Tolylsulfonyl)hydrazon]-LL-F28249α;
23-(4-Phenylsemicarbazon)-LL-F28249α;
23-[(2,3,4,5,6-Pentafluorphenyl)hydrazon]-LL-F28249α;
23-Oxamoylhydrazon-LL-F28249α;
23-[o-(Phenoxyacetoxy)oxim]-LL-F28249α;
23-[o-(Ethoxymethyl)oxim]-LL-F28249α;
23-o-Methyloxim-5-(phenoxyacetoxy)-LL-F28249α;
23-(4-Phenyl-3-thiosemicarbazon)-LL-F28249α;
5-O-t-Butyldimethylsilyl-23-oxim-LL-F28249α;
5-O-Trimethylsilyl-23-oxim-LL-F28249α;
oder die β-, γ-, δ-, ε-, ζ-, θ-, ι- und λ-Derivate jeder der obigen Silyl-Verbindungen.

7. Verfahren zur topischen oder systemischen Kontrolle von Pflanzeninsekten und zum Schutz von Feldfrüchten, Bäumen, Sträuchern, gelagertem Korn und Zierpflanzen, wobei das Verfahren gekennzeichnet ist durch:
Anwenden einer insektizid wirksamen Menge der Verbindung, die durch die Strukturformel (I) dargestellt wird,

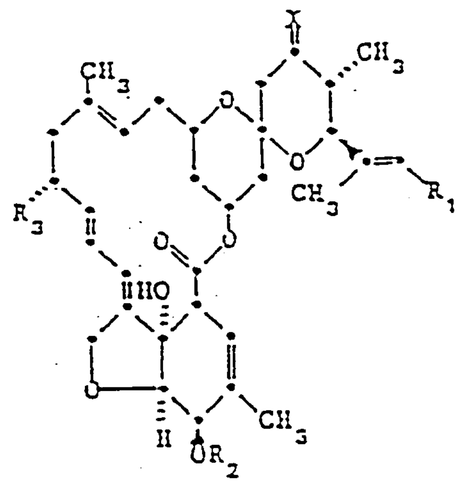

I

in der $R_1$, $R_2$, $R_3$ und X wie in Anspruch 1 definiert sind.

**8.** Verfahren nach Anspruch 7, worin die Verbindung mit $R_1$ als Isopropyl; $R_2$ als Wasserstoff; $R_3$ als Methyl und X als $NOCH_3$ vorliegt.

**9.** Verfahren zur Kontrolle von Pflanzennematoden, wobei das Verfahren gekennzeichnet ist durch:
Auftragen einer nematizid wirksamen Menge der Verbindung, die durch die Strukturformel (I) dargestellt wird,

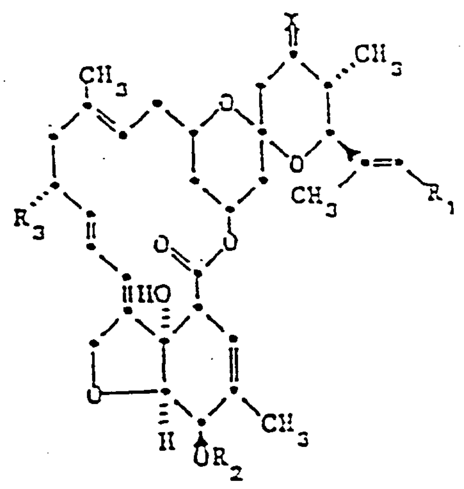

I

in der $R_1$, $R_2$, $R_3$ und X wie in Anspruch 1 definiert sind, auf die Blätter von Pflanzen, den Boden, in dem sie wachsen, oder in deren Stämme.

**10.** Verfahren nach Anspruch 9, in dem die Verbindung mit $R_1$ als Isopropyl; $R_2$ als Wasserstoff; $R_3$ als Methyl; und X als $NOCH_3$ vorliegt.

## Revendications

**Revendications pour les Etats contractants suivants : BE, CH, DE, FR, GB, IT, LI, LU, NL, SE**

**1.** Composés caractérisés par la formule développée (I) :

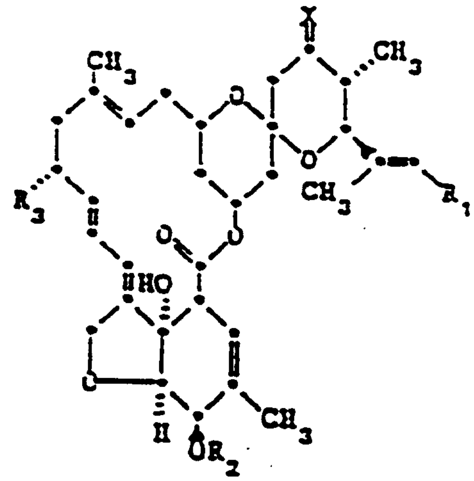

I

dans laquelle $R_1$ représente un groupe méthyle, éthyle ou isopropyle ; $R_2$ représente un atome d'hydrogène, un groupe méthyle, alcanoyle en $C_1$-$C_4$, méthoxyacétyle, chloroacétyle, dichloroacétyle, trichloroacétyle, trifluoroacétyle ; $R_3$ représente un atome d'hydrogène ou un groupe méthyle ; X représente $NOR_4$ ou $N$-$NHR_5$ ; $R_4$ représente un atome d'hydrogène, un groupe alkyle en $C_1$-$C_6$, benzyle, allyle, propargyle, phényle, $CH_2$-COO-(alkyle en $C_1$-$C_4$), N-(alkyl en $C_1$-$C_6$)carbamoyle, N-(allyl)carbamoyle, N-(propargyl)carbamoyle, N-(phényl)carbamoyle, N-(chlorophényl)carbamoyle, N-(dichlorophényl)carbamoyle, N-(benzyl)carbamoyle, alcanoyle en $C_1$-$C_6$, chloroacétyle, méthoxyacétyle, phénylacétyle ou chlorobenzoyle ; $R_5$ représente un groupe alcanoyle en $C_1$-$C_6$, formyle, alkyle en $C_1$-$C_6$, benzoyle éventuellement substitué avec un ou deux atomes d'halogène, groupes alkyle en $C_1$-$C_4$, groupes alkoxy en $C_1$-$C_4$ ou groupes nitro ;

et leurs sels pharmaceutiquement et pharmacologiquement acceptables.

**2.** Composé selon la revendication 1, dans lequel $R_1$ représente un groupe isopropyle ; $R_2$ représente un atome d'hydrogène ou un groupe méthyle ; $R_3$ représente un groupe méthyle ; et X représente $NOR_4$ ; dans lequel $R_1$ représente un groupe isopropyle ; $R_2$ représente un atome d'hydrogène ; $R_3$ représente un groupe méthyle ; X représente $NOR_4$, $N$-$NHCH_3$, et $R_4$ représente un groupe alkyle en $C_1$-$C_3$, N-(alkyl en $C_1$-$C_6$)carbamoyle, N-(allyl)carbamoyle, N-(propargyl)carbamoyle, N-(phényl)carbamoyle, N-(4-chlorophényl)carbamoyle, N-(benzyl)carbamoyle, alcanoyle en $C_1$-$C_6$, chloroacétyle, méthoxyacétyle, benzoyle ou chlorobenzoyle ; dans lequel $R_1$ représente un groupe isopropyle ; $R_2$ représente un atome d'hydrogène ; $R_3$ représente un groupe méthyle ; X représente $NOR_4$ ; et $R_4$ représente un groupe méthyle, éthyle, N-(alkyl en $C_1$-$C_4$)carbamoyle, N-(phényl)carbamoyle, ou N-(4-chlorophényl)-carbamoyle ; dans lequel $R_1$ représente un groupe isopropyle ; $R_2$ représente un atome d'hydrogène ; $R_3$ représente un groupe méthyle ; et X représente $NOCH_3$ ; et dans lequel $R_1$ représente un groupe isopropyle ; $R_2$ représente un atome d'hydrogène ; $R_3$ représente un groupe méthyle ; et X représente

$$NNH-\overset{\overset{O}{\|}}{C}-NH_2.$$

**3.** Procédé de lutte topique ou systémique contre des insectes de plante, et de protection de cultures, d'arbres, d'arbustes, de grains conservés et de plantes d'ornement, selon lequel on applique une quantité à effet insecticide du composé représenté par la formule développée (I)

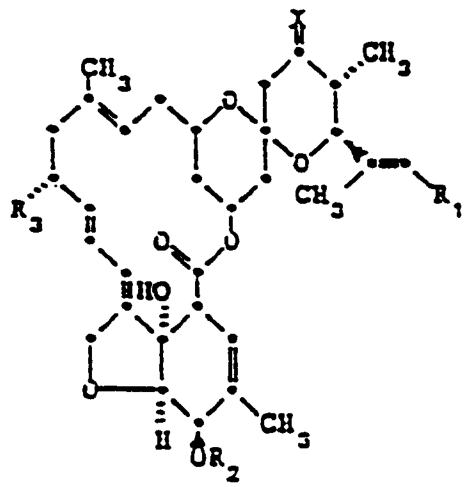

I

dans laquelle $R_1$, $R_2$, $R_3$ et X sont tels que définis dans la revendication 1.

**4.** Procédé selon la revendication 3, dans lequel dans le composé, $R_1$ représente un groupe isopropyle ; $R_2$ représente un atome d'hydrogène ; $R_3$ représente un groupe méthyle et X représente $NOCH_3$.

**5.** Procédé de lutte contre des nématodes de plante, selon lequel on applique sur le feuillage des plantes, le sol sur lequel elles sont cultivées ou dans leurs troncs, une quantité à effet nématicide du composé représenté par la formule développée (I) :

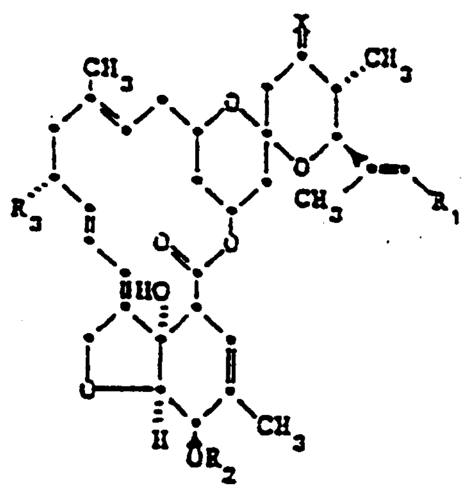

I

dans laquelle $R_1$, $R_2$, $R_3$ et X sont tels que définis dans la revendication 1.

**6.** Procédé selon la revendication 5, dans lequel dans le composé, $R_1$ représente un groupe isopropyle ; $R_2$ représente un atome d'hydrogène ; $R_3$ représente un groupe méthyle ; et X représente $NOCH_3$.

**7.** Composition de traitement, de prévention ou de lutte contre des infestations endo et/ou ectoparasitaires chez des animaux à sang chaud, caractérisée en ce qu'elle comprend une quantité à effet prophylactique, thérapeutique ou pharmaceutique du composé représenté par la formule développée (I) :

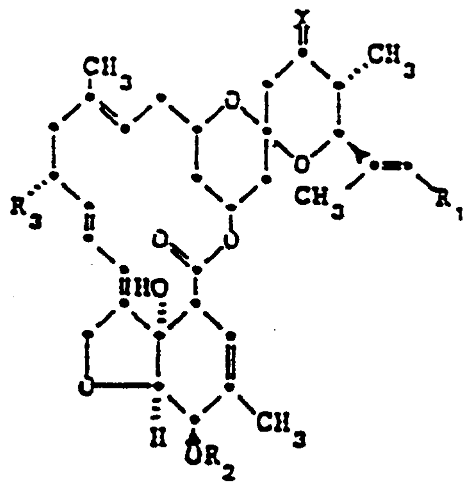

I

dans laquelle $R_1$, $R_2$, $R_3$ et X sont tels que définis dans la revendication 1.

**8.** Composition pour lutter contre des insectes, caractérisée en ce qu'elle comprend une quantité à effet insecticide du composé représenté par la formule développe (I) :

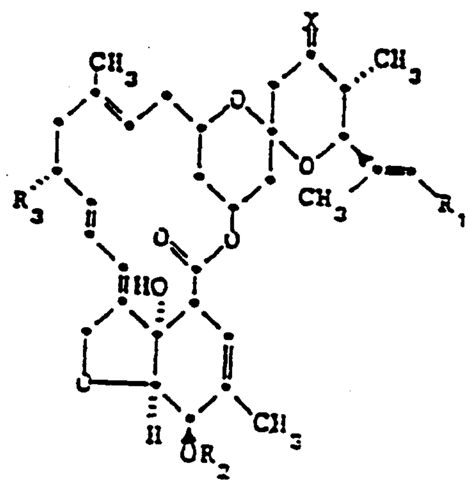

I

dans laquelle $R_1$, $R_2$, $R_3$ et X sont tels que définis dans la revendication 1.

**9.** Composé caractérisé en ce qu'il s'agit du 23-(formylhydrazone)-LL-F28249α ; du 23-[(tolylsulfonyl)-hydrazone]-LL-F28249α ; du 23-(4-phénylsemicarbazonne)-LL-F28249α ; du 23-[(2,3,4,5,6-pentafluorophényl)hydrazone]-LL-F28249α ; du 23-oxamoylhydrazone-LL-F28249α ; du 23-[o-phénoxyacétoxy)-oxime]-LL-F28249α ; du 23-[o-(éthoxyméthyl)oxime]-LL-F28249α ; du 23-o-méthyloxime-5-(phénoxyacétoxy)-LL-F28249α ; du 23-(4-phényl-3-thiosemicarbazone)-LL-F28249α ; du 5-O-t-butyldiméthylsilyl-23-oxime-LL-F28249α ; et du 5-O-triméthylsilyl-23-oxime-LL-F28249α ; ou les dérivés β, γ, δ, ε, ζ, θ, ι et λ de chacun des composés silylés ci-dessus.

**10.** Procédé de préparation d'un composé selon la revendication 1, selon lequel on prépare un 5-O-(trisilyl substitué)-LL-F28249α, β, γ, δ, ε, ζ, θ, ι , ou λ, ou un 5-O-(silyl trisubstitué) oxyacétyle -LL-F28249α, β, γ, δ, ε, ζ, θ, ι ou λ de structure :

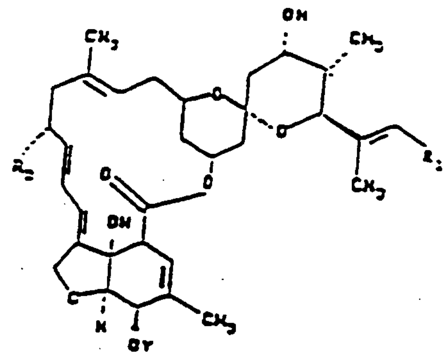

dans laquelle $R_1$ représente un groupe méthyle, éthyle ou isopropyle ; $R_3$ représente un atome d'hydrogène ou un groupe méthyle ; Y représente un groupe silyle trisubstitué ou une fonction 5-(silyl trisubstitué) oxyacétyle notamment un groupe t-butyldiméthylsilyle, triméthylsilyle ou t-butyldiméthylsilyloxyacétyle ; caractérisé en ce qu'on fait réagir un composé de structure :

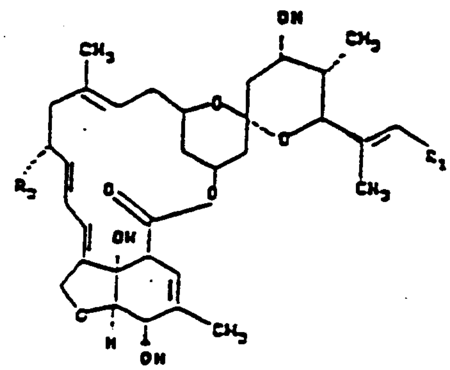

dans laquelle $R_1$ et $R_3$ sont tels que décrits ci-dessus, avec un halogénure de silyle trisubstitué ou un halogénure de (silyl trisubstitué) oxyacétyle, en présence d'une base organique et d'un solvant aprotique, à une température d'environ 0 °C à 30 °C, de sorte que l'on obtient le 5-O-(silyl trisubstitué)-LL-F28249α, β, γ, δ, ε, ζ, θ, ι ou λ, ou le 5-O-(silyl trisubstitué) oxyacétyle-LL-F28249α, β, γ, δ, ε, ζ, θ, ι et λ requis.

**11.** Procédé selon la revendication 10, dans lequel la base organique est l'imidazole, la pyridine, la 4-diméthylaminopyridine ou la triéthylamine ; le solvant aprotique et le chlorure de méthylène, le toluène, l'acétate d'éthyle, le tétrahydrofuranne ou le chlorure d'éthylène, et la fonction silyle trisubstituée est le groupe t-butyldiméthylsilyle, triméthylsilyle ou t-butyldiméthylsilyloxyacétyle.

**12.** Procédé de préparation d'un composé selon la revendication 1, selon lequel on prépare un 5-O-(silyl trisubstitué)-23-oxo-LL-F28249α, β, γ, δ, ε, ζ, θ, ι et λ de structure :

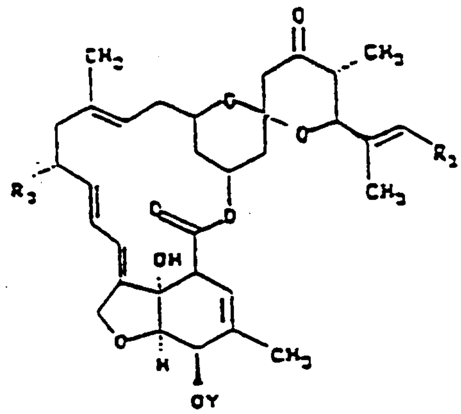

dans laquelle $R_1$ représente un groupe méthyle, éthyle ou isopropyle ; $R_3$ représente un atome d'hydrogène ou le groupe méthyle et Y représente une fonction silyle trisubstituée ou (silyl trisubstitué) oxyacétyle telle qu'un groupe t-butyldiméthylsilyle, triméthylsilyle ou t-butyldiméthylsilyloxyacétyle ; caractérisé en ce qu'on fait réagir un composé de structure :

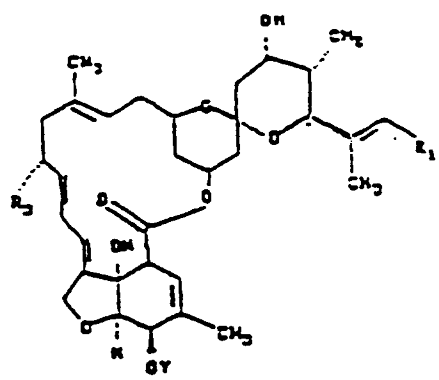

dans laquelle $R_1$, $R_3$ et Y sont tels que décrits ci-dessus, avec un agent oxydant tel que le dichromate de pyridinium, le chlorochromate de pyridinium, l'acide chromique-diméthylpyrazole, l'anhydride acétique/diméthylsulfoxyde, le N-chlorosuccinimide/diméthylsulfoxyde, le chlorure d'oxalyle/diméthylsulfoxyde ; en présence d'un solvant aprotique et éventuellement d'une base organique, à une température de -78 °C à +25 °C.

**13.** Procédé selon la revendication 12, dans lequel la base organique est la triéthylamine ou la diisopropyléthylamine ; le solvant aprotique est le chlorure de méthylène, le chlorure d'éthylène, le diméthylformamide ou le diméthylsulfoxyde ; de sorte que le 5-O-(silyl trisubstitué)-23-oxo-LL-F28249$\alpha$, $\beta$, $\gamma$, $\delta$, $\epsilon$, $\zeta$, $\theta$, $\iota$ ou $\lambda$, le 5-O-(silyl trisubstitué) oxyacétyle -23-oxo-LL-F28249$\alpha$, $\beta$, $\gamma$, $\delta$, $\epsilon$, $\zeta$, $\theta$, $\iota$ ou $\lambda$ requis, est obtenu.

**14.** Composé caractérisé en ce qu'il s'agit du 5-O-t-butyldiméthylsilyl-LL-F28249$\alpha$ ; du 5-O-triméthylsilyl-LL-F28249$\alpha$ ; du 5-O-t-butyldiméthylsilyl-23-oxo-LL-F28249$\alpha$ ; du 5-O-t-triméthylsilyl-23-oxo-LL-F28249$\alpha$ ; et du 5-O-t-butyldiméthylsilyloxyacétyl-LL-F28249$\alpha$, ou des dérivés $\beta$, $\gamma$, $\delta$, $\epsilon$, $\zeta$, $\theta$, $\iota$ ou $\lambda$ de chacun des composés silylés ci-dessus.

**Revendications pour les Etats contractants suivants : AT, ES, GR**

**1.** Procédé de préparation d'un composé caractérisé par la formule développée (I) :

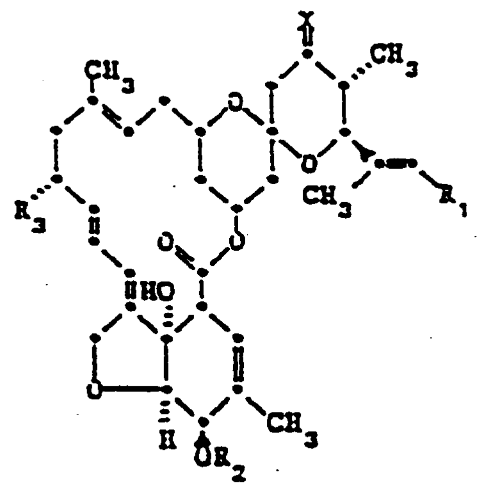

I

dans laquelle $R_1$ représente un groupe méthyle, éthyle ou isopropyle ; $R_2$ représente un atome d'hydrogène, un groupe méthyle, alcanoyle en $C_1$-$C_4$, méthoxyacétyle, chloroacétyle, dichloroacétyle, trichloroacétyle, trifluoroacétyle ; $R_3$ représente un atome d'hydrogène ou un groupe méthyle ; X représente $NOR_4$ ou $N$-$NHR_5$ ; $R_4$ représente un atome d'hydrogène, un groupe alkyle en $C_1$-$C_6$, benzyle, allyle, propargyle, phényle, $CH_2$-COO-(alkyle en $C_1$-$C_4$), N-(alkyl en $C_1$-$C_6$)carbamoyle, N-(allyl)carbamoyle, N-(propargyl)carbamoyle, N-(phényl)carbamoyle, N-(chlorophényl)carbamoyle, N-(dichlorophényl)carbamoyle, N-(benzyl)carbamoyle, alcanoyle en $C_1$-$C_6$, chloroacétyle, méthoxyacétyle, phénylacétyle ou chlorobenzoyle ; $R_5$ représente un groupe alcanoyle en $C_1$-$C_6$, formyle, alkyle en $C_1$-$C_6$, benzoyle éventuellement substitué avec un ou deux atomes d'halogène, groupes alkyle en $C_1$-$C_4$, groupes alkoxy en $C_1$-$C_4$ ou groupes nitro ; et ses sels pharmaceutiquement et pharmacologiquement acceptables, selon lequel on prépare un 5-O-(silyl trisubstitué)-LL-F28249$\alpha$, $\beta$, $\gamma$, $\delta$, $\epsilon$, $\zeta$, $\theta$, $\iota$ ou $\lambda$, ou un 5-O-(silyl trisubstitué) oxyacétyle -LL-F28249$\alpha$, $\beta$, $\gamma$, $\delta$, $\epsilon$, $\zeta$, $\theta$, $\iota$ ou $\lambda$ de structure :

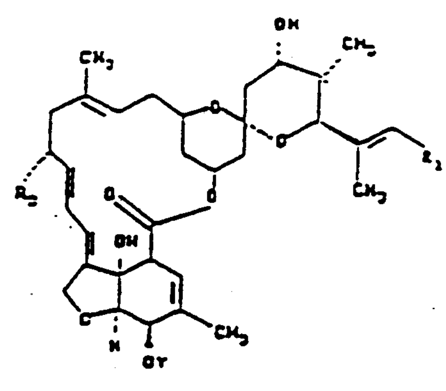

dans laquelle $R_1$ représente un groupe méthyle, éthyle ou isopropyle ; $R_3$ représente un atome d'hydrogène ou un groupe méthyle ; Y représente une fonction silyle trisubstituée ou 5-silyl trisubstitué) oxyacétyle-LL-notamment un groupe t-butyldiméthylsilyle, triméthylsilyle ou t-butyldiméthylsilyloxyacétyle ; caractérisé en ce qu'on fait réagir un composé de structure :

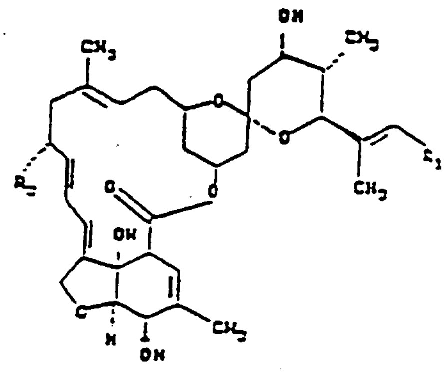

dans laquelle $R_1$ et $R_3$ sont tels que décrits ci-dessus, avec un halogénure de silyle trisubstitué ou un halogénure de(silyl trisubstitué) oxyacétyle, en présence d'une base organique et d'un solvant aprotique, à une température d'environ 0 °C à 30 °C, de sorte que l'on obtient le 5-O-(silyl trisubstitué)-LL-F28249α, β, γ, δ, ε, ζ, θ, ι ou λ, ou le 5-O-(silyl trisubstitué) oxyacétyle-LL-F28249α, β, γ, δ, ε, ζ, θ, ι ou λ, requis.

2. Procédé selon la revendication 1, dans lequel la base organique est l'imidazole, la pyridine, la 4-diméthylaminopyridine ou la triéthylamine ; le solvant aprotique est le chlorure de méthylène, le toluène, l'acétate d'éthyle, le tétrahydrofuranne ou le chlorure d'éthylène, et la fonction silyle trisubstituée, est un groupe t-butyldiméthylsilyle, triméthylsilyle ou t-butyldiméthylsilyloxyacétyle.

3. Procédé de préparation d'un composé selon la revendication 1, dans lequel on prépare un 5-O-(silyl trisubstitué)-23-oxo-LL-F28249α, β, γ, δ, ε, ζ, θ, ι et λ de structure :

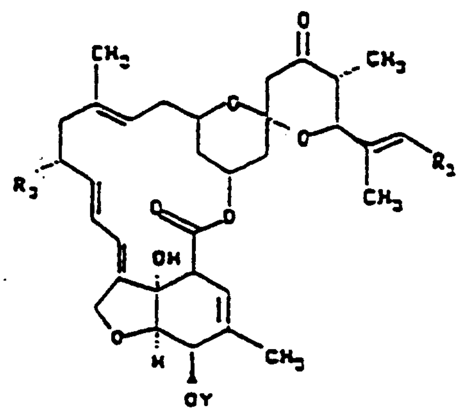

dans laquelle $R_1$ représente un groupe méthyle, éthyle ou isopropyle ; $R_3$ représente un atome d'hydrogène ou un groupe méthyle et Y représente une fonction silyle trisubstituée ou ( silyl trisubstitué) oxyacétyle, notamment un groupe t-butyldiméthylsilyle, triméthylsilyle ou t-butyldiméthylsilyloxyacétyle ; caractérisé en ce qu'on fait réagir un composé de structure :

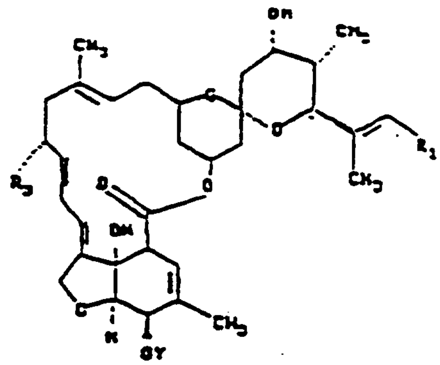

dans laquelle $R_1$, $R_3$ et Y sont tels que définis ci-dessus, avec un agent oxydant tel que le dichromate de pyridinium, le chlorochromate de pyridinium, l'acide chromique-diméthylpyrazole, l'anhydride acétique/diméthylsulfoxyde, le N-chlorosuccinimide/diméthylsulfoxyde, le chlorure d'oxalyle/diméthylsulfoxyde ; en présence d'un solvant aprotique et éventuellement d'une base organique, à une température de -78 °C à +25 °C.

4. Procédé selon la revendication 3, dans lequel la base organique est la triéthylamine ou la diisopropyléthylamine ; le solvant aprotique est le chlorure de méthylène, le chlorure d'éthylène, le diméthylformamide ou le diméthylsulfoxyde ; de sorte que l'on obtient le 5-O-(silyl trisubstitué)-23-oxo-LL-F28249$\alpha$,$\beta$, $\gamma$, $\delta$, $\epsilon$, $\zeta$, $\theta$, $\iota$ ou $\lambda$ le 5-O-(silyl trisubstitué) oxyacétyle-23-oxo-LL-F28249$\alpha$, $\beta$, $\gamma$, $\delta$, $\epsilon$, $\zeta$, $\theta$, $\iota$ ou $\lambda$ requis.

5. Procédé selon la revendication 1, dans lequel $R_1$ représente un groupe isopropyle ; $R_2$ représente un atome d'hydrogène ou un groupe méthyle ; $R_3$ représente un groupe méthyle ; et X représente $NOR_4$ ; dans lequel $R_1$ représente un groupe isopropyle ; $R_2$ représente un atome d'hydrogène ; $R_3$ représente un groupe méthyle ; X représente $NOR_4$, N-$NHCH_3$, et $R_4$ représente un groupe alkyle en $C_1$-$C_3$, N-(alkyl en $C_1$-$C_6$)carbamoyle, N-(allyl)carbamoyle, N-(propargyl)carbamoyle, N-(phényl)carbamoyle, N-(4-chlorophényl)carbamoyle, N-(benzyl)carbamoyle, alcanoyle en $C_1$-$C_6$, chloroacétyle, méthoxyacétyle, benzoyle ou chlorobenzoyle ; dans lequel $R_1$ représente un groupe isopropyle ; $R_2$ représente un atome d'hydrogène ; $R_3$ représente un groupe méthyle ; X représente $NOR_4$ ; et $R_4$ représente un groupe méthyle, éthyle, N-(alkyl en $C_1$-$C_4$)carbamoyle, N-(phényl)carbamoyle, ou N-(4-chlorophényl)-carbamoyle ; dans lequel $R_1$ représente un groupe isopropyle ; $R_2$ représente un atome d'hydrogène ; $R_3$ représente un groupe méthyle ; et X représente $NOCH_3$ ; et dans lequel $R_1$ représente un groupe isopropyle ; $R_2$ représente un atome d'hydrogène ; $R_3$ représente un groupe méthyle ; et X représente

$$NNH-\overset{O}{\overset{\|}{C}}-NH_2.$$

6. Procédé selon la revendication 1, pour la production du 23-(formylhydrazone)-LL-F28249$\alpha$ ; du 23-[-(tolylsulfonyl)hydrazone]-LL-F28249$\alpha$ ; du 23-(4-phénylsemicarbazone )-LL-F28249$\alpha$ ; du 23-[(2,3,4,5,6-pentafluorophényl)hydrazone]-LL-F28249$\alpha$ ; du 23-oxamoylhydrazone-LL-F28249$\alpha$ ; du 23-[o-phénoxyacétoxy)oxime]-LL-F28249$\alpha$ ; du 23-[o-(éthoxyméthyl)oxime]-LL-F28249$\alpha$ ; du 23-o-méthyloxime-5-(phénoxyacétoxy)-LL-F28249$\alpha$ ; du 23-(4-phényl-3-thiosemicarbazone)-LL-F28249$\alpha$ ; du 5-O-t-butyldiméthylsilyl-23-oxime-LL-F28249$\alpha$ ; et du 5-O-triméthylsilyl-23-oxime-LL-F28249$\alpha$ ; ou les dérivés $\beta$, $\gamma$, $\delta$, $\epsilon$, $\zeta$, $\theta$, $\iota$ ou $\lambda$ de chacun de ces composés silylés ci-dessus.

7. Procédé de lutte topique ou systémique contre des insectes de plante, et de protection de cultures, d'arbres, d'arbustes, de grains conservés et de plantes d'ornement, selon lequel on applique une quantité à effet insecticide du composé représenté par la formule développée (I)

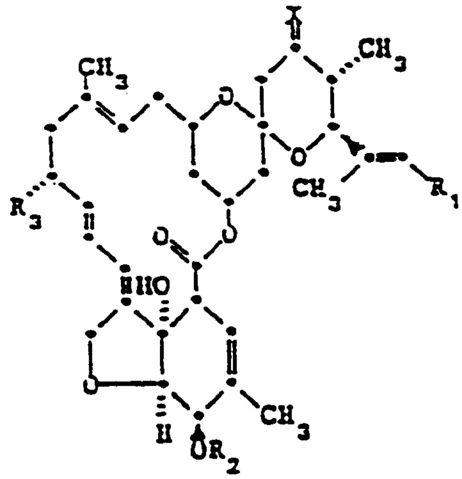

I

dans laquelle $R_1$, $R_2$, $R_3$ et X sont tels que définis dans la revendication 1.

**8.** Procédé selon la revendication 7, dans lequel dans le composé, $R_1$ représente un groupe isopropyle ; $R_2$ représente un atome d'hydrogène ; $R_3$ représente un groupe méthyle et X représente $NOCH_3$.

**9.** Procédé de lutte contre des nématodes de plante , selon lequel on applique sur le feuillage des plantes, le sol sur lequel elles sont cultivées ou dans leurs troncs, une quantité à effet nématicide du composé représenté par la formule développée (I) :

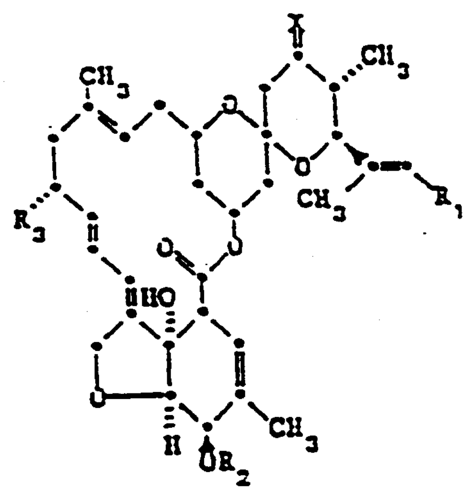

I

dans laquelle $R_1$, $R_2$, $R_3$ et X sont tels que définis dans la revendication 1.

**10.** Procédé selon la revendication 9, dans lequel dans le composé, $R_1$ représente un groupe isopropyle ; $R_2$ représente un atome d'hydrogène ; $R_3$ représente un groupe méthyle ; et X représente $NOCH_3$.